# EUROPEAN PATENT APPLICATION

(11) **EP 4 233 924 A1**
(43) Date of publication of application: **30.08.2023**
(21) Application number: 21898699.0
(22) Date of filing: 29.11.2021
(51) Int. Cl.: A61L 27/36, A61L 27/38, A61L 27/24, A61F 2/08

(54) **TISSUE GEL FOR TRANSPLANTATION USING DECELLULARIZED EXTRACELLULAR MATRIX AND METHOD FOR PRODUCING SAME**

(30) Priority: 27.11.2020 KR 20200163266
(71) Applicant: Cellartgen Inc., Seoul 03722 (KR)
(72) Inventor: CHO, Seung Woo, Seoul 03722 (KR); CHOI, Nak Won, Seoul 02792 (KR); JIN, Yoon Hee, Seoul 03722 (KR); JEON, Eun Je, Seoul 03722 (KR); CHOI, Yi Sun, Seoul 03722 (KR)
(74) Representative: Manitz Finsterwald Patent- und Rechtsanwaltspartnerschaft mbB
(86) International application number: PCT/KR2021/017697
(87) International publication number: WO 2022/114874

(57) **Abstract**

The present disclosure relates to a tissue gel for transplantation and a method of preparing the same, including: a process (a) of preparing a composition containing a decellularized extracellular matrix; a process (b) of maintaining a tensile state by applying a tensile force to a stretching device made of an elastic polymer material; and a process (c) of injecting the composition prepared in the process (a) into the stretching device maintained in the tensile state in the process (b), allowing the composition to crosslink, and then removing the tensile force to align fibrils in the decellularized extracellular matrix in one direction.

## Description

### TECHNICAL FIELD

The present disclosure relates to a tissue-derived gel for transplantation using a decellularized extracellular matrix (particularly, decellularized skeletal muscle extracellular matrix) and a method of preparing the same.

### BACKGROUND

Although the number of patients with intractable muscular disorders caused by traumatic or hereditary muscle damage is rapidly increasing, there is currently no fundamental therapeutic method. In the recent aging society, sarcopenia caused by aging leads to a high mortality rate in the elderly.

In the case of traumatic muscle damage (e.g., accidental trauma, tumor removal surgery, etc.), it is treated by transplanting a muscle from elsewhere in the patient's body to a damaged site to reconstruct it. However, this approach can cause secondary damage and lead to reduced functionality of the transplanted tissue. There are ongoing efforts to develop gene therapy and cell therapy for hereditary muscle damage, but no successful clinical cases have been reported. Sarcopenia, a muscle loss condition that occurs with aging, has only recently been recognized as a disease with no established treatment. To overcome the limitations of conventional methods, urgent development of tissue engineering-based muscle regeneration treatments is needed.

Combination of muscle cells and polymeric scaffolds, which are commonly used in traditional tissue engineering methods, have not been successful in creating fully functional three-dimensional artificial muscle constructs. While 3D printing technology can produce muscle-like structures, achieving a precise muscle bundle arrangement in a three-dimension is still challenging.

Recently, a direct reprogramming technology, which induces direct conversion from somatic cells into other tissue-specific cells, has attracted a lot of attention as a technology for producing patient-specific cell therapeutic agents. However, the resultant muscle cells have very low differentiation potency and exhibit limited functionality, so it is difficult to expect muscle regenerative efficacy. No tissue engineering attempt has been made to improve the differentiation potency and functionality of induced skeletal myogenic progenitor cells (iMPCs) produced by direct reprogramming, and there is insufficient research applying the iMPCs to efficient muscle regeneration in vivo.

For efficient muscle regeneration, a scaffold that mimics a specific microstructure of a muscle bundle needs to be fabricated. Therefore, a technology for aligning cells in the form of muscle tissue needs to be developed. There have been developed some technologies, e.g., 3D printing and electrospinning, for inducing cell alignment in a three-dimensional environment based on a synthetic polymer or a single extracellular matrix (e.g., collagen) component. However, according to the conventional methods, it is impossible to mimic complex extracellular matrix components present in the muscle tissue, and, thus, there are limitations in inducing differentiation of cells, enhancing a survival rate, and maintaining plasma for a long time.

### DISCLOSURE OF THE INVENTION

### PROBLEMS TO BE SOLVED BY THE INVENTION

The present disclosure is conceived to align fibrils in a decellularized extracellular matrix in one direction and provide a method of preparing a tissue gel for transplantation, including: a process (a) of preparing a composition containing the extracellular matrix; a process (b) of maintaining a tensile state by applying a tensile force to a stretching device made of an elastic polymer material; and a process (c) of injecting the composition prepared in the process (a) into the stretching device maintained in the tensile state in the process (b), allowing the composition to crosslink, and then removing the tensile force.

However, the problems to be solved by the present disclosure are not limited to the above-described problems. Although not described herein, other problems to be solved by the present disclosure can be clearly understood by a person with ordinary skill in the art from the following descriptions.

### MEANS FOR SOLVING THE PROBLEMS

An aspect of the present disclosure provides a method of preparing a tissue gel for transplantation, including: a process (a) of preparing a composition containing a decellularized extracellular matrix; a process (b) of maintaining a tensile state by applying a tensile force to a stretching device made of an elastic polymer material; and a process (c) of injecting the composition prepared in the process (a) into the stretching device maintained in the tensile state in the process (b), allowing the composition to crosslink, and then removing the tensile force to align fibrils in the decellularized extracellular matrix in one direction.

In an embodiment of the present disclosure, in the process (a), the decellularized extracellular matrix is derived from one or more tissues selected from the group consisting of muscle, brain, spinal cord, tongue, airway, skin, lymph, lung, heart, liver, stomach, kidney, pancreas, intestine, skeletal muscle, fat, uterus, thymus, esophagus, salivary gland, bone, bladder, blood vessel, tendon, pancreas, thyroid gland, ligament and cartilage.

In an embodiment of the present disclosure, in the process (a), the decellularized extracellular matrix includes collagen, glycoprotein, proteoglycan, ECM-affiliated protein and ECM modulator, and the collagen, glycoprotein, proteoglycan, ECM-affiliated protein and ECM modulator are included in an amount of 15 wt% to 45 wt%, 20 wt% to 40 wt%, 10 wt% to 20 wt%, 0.1 wt% to 10 wt% and 10 wt% to 30 wt%, respectively, with respect to a total weight of the decellularized extracellular matrix protein.

In an embodiment of the present disclosure, the process (a) further includes loading induced skeletal myogenic progenitor cells or induced neuronal cells into the composition.

In an embodiment of the present disclosure, in the process (b), the stretching device is an elastic material containing polydimethylsiloxane (PDMS).

In an embodiment of the present disclosure, in the process (a), the tensile force acts in a direction perpendicular to the one direction of the process (c).

In an embodiment of the present disclosure, the preparation method may further include a process (d) of culturing the loaded induced skeletal myogenic progenitor cells or induced neuronal cells after the tensile force is removed in the process (c).

In an embodiment of the present disclosure, in the process (d), the culture may be performed within 21 days at a temperature of 30°C to 40°C.

Another aspect of the present disclosure provides a tissue gel for transplantation including a decellularized extracellular matrix which includes collagen, glycoprotein, proteoglycan, ECM-affiliated protein and ECM modulator and in which the collagen, glycoprotein, proteoglycan, ECM-affiliated protein and ECM modulator are included in an amount of 15 wt% to 45 wt%, 20 wt% to 40 wt%, 10 wt% to 20 wt%, 0.1 wt% to 10 wt% and 10 wt% to 30 wt%, respectively, with respect to a total weight of the decellularized extracellular matrix protein, and fibrils in the decellularized extracellular matrix are aligned in one direction.

In an embodiment of the present disclosure, the decellularized extracellular matrix may be derived from one or more tissues selected from the group consisting of muscle, brain, spinal cord, tongue, airway, skin, lymph, lung, heart, liver, stomach, kidney, pancreas, intestine, skeletal muscle, fat, uterus, thymus, esophagus, salivary gland, bone, bladder, blood vessel, tendon, pancreas, thyroid gland, ligament and cartilage.

In an embodiment of the present disclosure, the tissue gel for transplantation further includes induced skeletal myogenic progenitor cells or induced neuronal cells loaded and cultured in the decellularized extracellular matrix.

In an embodiment of the present disclosure, as a result of measuring a storage modulus of the tissue gel for transplantation, the storage modulus is from 300 Pa to 1,000 Pa.

### EFFECTS OF THE INVENTION

According to the present disclosure, a tensile force is applied to a decellularized extracellular matrix in the other direction (perpendicular to one direction) and then removed to align fibrils in the decellularized extracellular matrix in the one direction. Thus, it is possible to effectively implement a tissue-specific microstructure. Therefore, a tissue gel for transplantation prepared according to the present disclosure can be applied not only to an artificial muscle tissue, but also to a technology for regeneration of various tissues having fibril alignment and orientation.

Also, the tissue gel for transplantation prepared according to the present disclosure can lyophilized for a long time. Thus, it can be easily stored and can be processed into various forms such as a hydrogel and a patch. Therefore, the tissue gel for transplantation can be widely applied to tissue engineering and regenerative medicine.

In particular, a muscle tissue gel for transplantation prepared according to the present disclosure is expected to be applied as a therapeutic agent for various muscle diseases, such as traumatic muscle damage, Duchenne muscular dystrophy, muscular atrophy, and sarcopenia caused by aging, which are creating a huge market worldwide. Further, the muscle tissue gel for transplantation is expected to be highly used as an in vitro model such as a muscle disease model and a toxicity evaluation and drug screening platform.

### BRIEF DESCRIPTION OF THE DRAWINGS

**FIG.** 1 shows fabrication of a tissue-engineered artificial muscle through three-dimensional alignment of a decellularized skeletal muscle extracellular matrix and induced skeletal myogenic progenitor cells.
**FIG. 2a** shows an analysis of the decellularized skeletal muscle extracellular matrix and induction of three-dimensional anisotropic alignment of the extracellular matrix.
**FIG. 2b** shows an analysis of the decellularized skeletal muscle extracellular matrix and induction of three-dimensional anisotropic alignment of the extracellular matrix.
**FIG. 2c** shows an analysis of the decellularized skeletal muscle extracellular matrix and induction of three-dimensional anisotropic alignment of the extracellular matrix.
**FIG. 2d** shows an analysis of the decellularized skeletal muscle extracellular matrix and induction of three-dimensional anisotropic alignment of the extracellular matrix.
**FIG. 2e** shows an analysis of the decellularized skeletal muscle extracellular matrix and induction of three-dimensional anisotropic alignment of the extracellular matrix.
**FIG. 2f** shows an analysis of the decellularized skeletal muscle extracellular matrix and induction of three-dimensional anisotropic alignment of the extracellular matrix.
**FIG. 2g** shows an analysis of the decellularized skeletal muscle extracellular matrix and induction of three-dimensional anisotropic alignment of the extracellular matrix.
**FIG. 2h** shows an analysis of the decellularized skeletal muscle extracellular matrix and induction of three-dimensional anisotropic alignment of the extracellular matrix.
**FIG. 3a** shows the proteomics of the decellularized skeletal muscle extracellular matrix.
**FIG. 3b** shows the proteomics of the decellularized skeletal muscle extracellular matrix.
**FIG. 3c** shows the proteomics of the decellularized skeletal muscle extracellular matrix.
**FIG. 3d** shows the proteomics of the decellularized skeletal muscle extracellular matrix.
**FIG. 3e** shows the proteomics of the decellularized skeletal muscle extracellular matrix.
**FIG. 4a** shows an analysis of induced skeletal myogenic progenitor cells produced by using a direct reprogramming technology.
**FIG. 4b** shows an analysis of induced skeletal myogenic progenitor cells produced by using a direct reprogramming technology.
**FIG. 4c** shows an analysis of induced skeletal myogenic progenitor cells produced by using a direct reprogramming technology.
**FIG. 5** shows an analysis of viability of the iMPCs three-dimensionally aligned in an MEM hydrogel.
**FIG. 6a** shows an analysis of the iMPCs aligned in the MEM hydrogel.
**FIG. 6b** shows an analysis of the iMPCs aligned in the MEM hydrogel.
**FIG. 6c** shows an analysis of the iMPCs aligned in the MEM hydrogel.
**FIG. 6d** shows an analysis of the iMPCs aligned in the MEM hydrogel.
**FIG. 6e** shows an analysis of the iMPCs aligned in the MEM hydrogel.
**FIG. 7a** shows an analysis and assessment of the degree of differentiation of the iMPCs aligned in a 3D MEM hydrogel.
**FIG. 7b** shows an analysis and assessment of the degree of differentiation of the iMPCs aligned in a 3D MEM hydrogel.
**FIG. 7c** shows an analysis and assessment of the degree of differentiation of the iMPCs aligned in a 3D MEM hydrogel.
**FIG. 7d** shows an analysis and assessment of the degree of differentiation of the iMPCs aligned in a 3D MEM hydrogel.
**FIG. 7e** shows an analysis and assessment of the degree of differentiation of the iMPCs aligned in a 3D MEM hydrogel.
**FIG. 7f** shows an analysis and assessment of the degree of differentiation of the iMPCs aligned in a 3D MEM hydrogel.
**FIG. 8a** shows an analysis of a differentiation-related signaling mechanism of induced skeletal myogenic progenitor cells cultured in the 3D MEM hydrogel.
**FIG. 8b** shows an analysis of a differentiation-related signaling mechanism of induced skeletal myogenic progenitor cells cultured in the 3D MEM hydrogel.
**FIG. 8c** shows an analysis of a differentiation-related signaling mechanism of induced skeletal myogenic progenitor cells cultured in the 3D MEM hydrogel.
**FIG. 8d** shows an analysis of a differentiation-related signaling mechanism of induced skeletal myogenic progenitor cells cultured in the 3D MEM hydrogel.
**FIG. 8e** shows an analysis of a differentiation-related signaling mechanism of induced skeletal myogenic progenitor cells cultured in the 3D MEM hydrogel.
**FIG. 8f** shows an analysis of a differentiation-related signaling mechanism of induced skeletal myogenic progenitor cells cultured in the 3D MEM hydrogel.
**FIG. 9a** shows an analysis of epigenetic regulators in iMPCs cultured in the 3D MEM hydrogel.
**FIG. 9b** shows an analysis of epigenetic regulators in iMPCs cultured in the 3D MEM hydrogel.
**FIG. 9c** shows an analysis of epigenetic regulators in iMPCs cultured in the 3D MEM hydrogel.
**FIG. 9d** shows an analysis of epigenetic regulators in iMPCs cultured in the 3D MEM hydrogel.
**FIG. 9e** shows an analysis of epigenetic regulators in iMPCs cultured in the 3D MEM hydrogel.
**FIG. 10a** shows an analysis of mechanotransduction signaling in the iMPCs cultured in the 3D MEM hydrogel.
**FIG. 10b** shows an analysis of mechanotransduction signaling in the iMPCs cultured in the 3D MEM hydrogel.
**FIG. 10c** shows an analysis of mechanotransduction signaling in the iMPCs cultured in the 3D MEM hydrogel.
**FIG. 10d** shows an analysis of mechanotransduction signaling in the iMPCs cultured in the 3D MEM hydrogel.
**FIG. 10e** shows an analysis of mechanotransduction signaling in the iMPCs cultured in the 3D MEM hydrogel.
**FIG. 10f** shows an analysis of mechanotransduction signaling in the iMPCs cultured in the 3D MEM hydrogel.
**FIG. 11a** shows fabrication of a volumetric muscle loss (VML) mouse model.
**FIG. 11b** shows fabrication of a volumetric muscle loss (VML) mouse model.
**FIG. 12a** shows a histological analysis (weeks 1 and 4) after transplantation of an artificial muscle tissue into the volumetric muscle loss (VML) mouse model.
**FIG. 12b** shows a histological analysis (weeks 1 and 4) after transplantation of an artificial muscle tissue into the volumetric muscle loss (VML) mouse model.
**FIG. 12c** shows a histological analysis (weeks 1 and 4) after transplantation of an artificial muscle tissue into the volumetric muscle loss (VML) mouse model.
**FIG. 12d** shows a histological analysis (weeks 1 and 4) after transplantation of an artificial muscle tissue into the volumetric muscle loss (VML) mouse model.
**FIG. 12e** shows a histological analysis (weeks 1 and 4) after transplantation of an artificial muscle tissue into the volumetric muscle loss (VML) mouse model.
**FIG. 12f** shows a histological analysis (weeks 1 and 4) after transplantation of an artificial muscle tissue into the volumetric muscle loss (VML) mouse model.
**FIG. 12g** shows a histological analysis (weeks 1 and 4) after transplantation of an artificial muscle tissue into the volumetric muscle loss (VML) mouse model.
**FIG. 12h** shows a histological analysis (weeks 1 and 4) after transplantation of an artificial muscle tissue into the volumetric muscle loss (VML) mouse model.
**FIG. 13a** shows a histological analysis (week 7) and a motor ability/ behavior assessment after transplantation of the artificial muscle tissue into the volumetric muscle loss (VML) mouse model.
**FIG. 13b** shows a histological analysis (week 7) and a motor ability/ behavior assessment after transplantation of the artificial muscle tissue into the volumetric muscle loss (VML) mouse model.
**FIG. 13c** shows a histological analysis (week 7) and a motor ability/ behavior assessment after transplantation of the artificial muscle tissue into the volumetric muscle loss (VML) mouse model.
**FIG. 13d** shows a histological analysis (week 7) and a motor ability/ behavior assessment after transplantation of the artificial muscle tissue into the volumetric muscle loss (VML) mouse model.
**FIG. 13e** shows a histological analysis (week 7) and a motor ability/ behavior assessment after transplantation of the artificial muscle tissue into the volumetric muscle loss (VML) mouse model.
**FIG. 13f** shows a histological analysis (week 7) and a motor ability/ behavior assessment after transplantation of the artificial muscle tissue into the volumetric muscle loss (VML) mouse model.
**FIG. 13g** shows a histological analysis (week 7) and a motor ability/ behavior assessment after transplantation of the artificial muscle tissue into the volumetric muscle loss (VML) mouse model.
**FIG. 13h** shows a histological analysis (week 7) and a motor ability/ behavior assessment after transplantation of the artificial muscle tissue into the volumetric muscle loss (VML) mouse model.
**FIG. 14a** shows a histological analysis and a kinematic analysis and assessment after transplantation of the artificial muscle tissue into an MDX transgenic mouse model of Duchenne muscular dystrophy (DMD).
**FIG. 14b** shows a histological analysis and a kinematic analysis and assessment after transplantation of the artificial muscle tissue into an MDX transgenic mouse model of Duchenne muscular dystrophy (DMD).
**FIG. 14c** shows a histological analysis and a kinematic analysis and assessment after transplantation of the artificial muscle tissue into an MDX transgenic mouse model of Duchenne muscular dystrophy (DMD).
**FIG. 14d** shows a histological analysis and a kinematic analysis and assessment after transplantation of the artificial muscle tissue into an MDX transgenic mouse model of Duchenne muscular dystrophy (DMD).
**FIG. 14e** shows a histological analysis and a kinematic analysis and assessment after transplantation of the artificial muscle tissue into an MDX transgenic mouse model of Duchenne muscular dystrophy (DMD).
**FIG. 14f** shows a histological analysis and a kinematic analysis and assessment after transplantation of the artificial muscle tissue into an MDX transgenic mouse model of Duchenne muscular dystrophy (DMD).
**FIG. 14g** shows a histological analysis and a kinematic analysis and assessment after transplantation of the artificial muscle tissue into an MDX transgenic mouse model of Duchenne muscular dystrophy (DMD).
**FIG. 14h** shows a histological analysis and a kinematic analysis and assessment after transplantation of the artificial muscle tissue into an MDX transgenic mouse model of Duchenne muscular dystrophy (DMD).
**FIG. 15** shows three-dimensional anisotropic alignment of induced neuronal cells cultured in a 3D BEM hydrogel.
**FIG. 16a** shows three-dimensional anisotropic alignment of induced skeletal myogenic progenitor cells and induced neuronal cells co-cultured in a 3D MEM hydrogel.
**FIG. 16b** shows three-dimensional anisotropic alignment of induced skeletal myogenic progenitor cells and induced neuronal cells co-cultured in a 3D MEM hydrogel.
**FIG. 16c** shows three-dimensional anisotropic alignment of induced skeletal myogenic progenitor cells and induced neuronal cells co-cultured in a 3D MEM hydrogel.
**FIG. 16d** shows three-dimensional anisotropic alignment of induced skeletal myogenic progenitor cells and induced neuronal cells co-cultured in a 3D MEM hydrogel.
**FIG. 16e** shows three-dimensional anisotropic alignment of induced skeletal myogenic progenitor cells and induced neuronal cells co-cultured in a 3D MEM hydrogel.
**FIG. 16f** shows three-dimensional anisotropic alignment of induced skeletal myogenic progenitor cells and induced neuronal cells co-cultured in a 3D MEM hydrogel.
**FIG. 16g** shows three-dimensional anisotropic alignment of induced skeletal myogenic progenitor cells and induced neuronal cells co-cultured in a 3D MEM hydrogel.
**FIG. 16h** shows three-dimensional anisotropic alignment of induced skeletal myogenic progenitor cells and induced neuronal cells co-cultured in a 3D MEM hydrogel.

### BEST MODE FOR CARRYING OUT THE INVENTION

The present disclosure provides a method of preparing a tissue gel for transplantation, including: a process (a) of preparing a composition containing a decellularized extracellular matrix; a process (b) of maintaining a tensile state by applying a tensile force to a stretching device made of an elastic polymer material; and a process (c) of injecting the composition prepared in the process (a) into the stretching device maintained in the tensile state in the process (b), allowing the composition to crosslink, and then removing the tensile force to align fibrils in the decellularized extracellular matrix in one direction.

In the process (a), the decellularized extracellular matrix may be derived from one or more tissues selected from the group consisting of muscle, brain, spinal cord, tongue, airway, skin, lymph, lung, heart, liver, stomach, kidney, pancreas, intestine, skeletal muscle, fat, uterus, thymus, esophagus, salivary gland, bone, bladder, blood vessel, tendon, pancreas, thyroid gland, ligament and cartilage.

In the process (a), the decellularized extracellular matrix may include collagen, glycoprotein, proteoglycan, ECM-affiliated protein and ECM modulator, and the collagen, glycoprotein, proteoglycan, ECM-affiliated protein and ECM modulator may be included in an amount of 15 wt% to 45 wt%, 20 wt% to 40 wt%, 10 wt% to 20 wt%, 0.1 wt% to 10 wt% and 10 wt% to 30 wt%, respectively, with respect to a total weight of the decellularized extracellular matrix protein.

The process (a) may further include loading induced skeletal myogenic progenitor cells or induced neuronal cells into the composition.

In the process (b), the stretching device may be an elastic material containing polydimethylsiloxane (PDMS).

In the process (b), the tensile force may act in a direction perpendicular to the one direction of the process (c).

The preparation method may further include a process (d) of culturing the loaded induced skeletal myogenic progenitor cells or induced neuronal cells after the tensile force is removed in the process (c).

In the process (d), the culture may be performed within 21 days at a temperature of 30°C to 40°C.

In an embodiment of the present disclosure, there is provided a tissue gel for transplantation including a decellularized extracellular matrix which includes collagen, glycoprotein, proteoglycan, ECM-affiliated protein and ECM modulator and in which the collagen, glycoprotein, proteoglycan, ECM-affiliated protein and ECM modulator are included in an amount of 15 wt% to 45 wt%, 20 wt% to 40 wt%, 10 wt% to 20 wt%, 0.1 wt% to 10 wt% and 10 wt% to 30 wt%, respectively, with respect to a total weight of the decellularized extracellular matrix protein, and fibrils in the decellularized extracellular matrix are aligned in one direction.

The decellularized extracellular matrix may be derived from one or more tissues selected from the group consisting of muscle, brain, spinal cord, tongue, airway, skin, lymph, lung, heart, liver, stomach, kidney, pancreas, intestine, skeletal muscle, fat, uterus, thymus, esophagus, salivary gland, bone, bladder, blood vessel, tendon, pancreas, thyroid gland, ligament and cartilage.

The tissue gel for transplantation may further include induced skeletal myogenic progenitor cells or induced neuronal cells loaded and cultured in the decellularized extracellular matrix.

As a result of measuring a storage modulus of the tissue gel for transplantation, the storage modulus may be from 300 Pa to 1,000 Pa.

### MODE FOR CARRYING OUT THE INVENTION

As for a microstructure of the actual muscle tissue, each myofiber is formed into a long, cylindrical, multinucleated parallel bundle through differentiation and cell fusion of myoblasts during a development process, and has a highly systemized microstructure. Due to the structural characteristics of an extracellular matrix in the muscle tissue, fibrils are aligned in a specific direction, which is essential for generation of muscle contractility. Therefore, a muscle tissue gel for transplantation capable of mimicking this structure needs to be fabricated.

The present inventors induced unidirectional alignment of fibrils in a decellularized skeletal muscle extracellular matrix using a stretching device made of an elastic polymer material (specifically, polydimethylsiloxane (PDMS)), and cultured induced skeletal myogenic progenitor cells prepared by using a direct reprogramming technology to fabricate an artificial muscle tissue gel that mimics the microstructure of a muscle bundle. The present disclosure was completed by successfully developing a tissue engineering technology that enhances the regenerative efficacy of the iMPCs by mimicking a dynamic fibril formation process using the flexible mechanical properties of PDMS and the decellularized muscle tissue-derived extracellular matrix.

Hereinafter, the present disclosure will be described in detail.

### Method of preparing tissue gel for transplantation

The present disclosure provides a method of preparing a tissue gel for transplantation, including: a process (a) of preparing a composition containing a decellularized extracellular matrix; a process (b) of maintaining a tensile state by applying a tensile force to a stretching device made of an elastic polymer material; and a process (c) of injecting the composition prepared in the process (a) into the stretching device maintained in the tensile state in the process (b), allowing the composition to crosslink, and then removing the tensile force to align fibrils in the decellularized extracellular matrix in one direction.

First, the method of preparing a tissue gel for transplantation according to the present disclosure includes the process of preparing a composition containing a decellularized extracellular matrix [process (a)].

The decellularized extracellular matrix is a decellularized extracellular matrix protein (particularly, decellularized skeletal muscle extracellular matrix protein), and may include collagen, glycoprotein, proteoglycan, ECM-affiliated protein and ECM modulator. The decellularized skeletal muscle extracellular matrix protein may be included in an amount of 10 wt% to 40 wt%, preferably 15 wt% to 30 wt% with respect to all of proteins in the decellularized extracellular matrix (particularly, decellularized skeletal muscle extracellular matrix). These components can form fibrils. To be specific, the collagen, glycoprotein, proteoglycan, ECM-affiliated protein and ECM modulator may be included in an amount of desirably 15 wt% to 45 wt%, 20 wt% to 40 wt%, 10 wt% to 20 wt%, 0.1 wt% to 10 wt% and 10 wt% to 30 wt%, respectively, with respect to a total weight of the decellularized extracellular matrix protein, but the present disclosure is not limited thereto. The decellularized extracellular matrix is prepared through decellularization including SDS or Triton X-100 treatment, and has a particularly high content of COL6A2, LOX, FGA, COL4A2, KERA, ITIH5, VCAN, FBN1, COL12A1 and LAMA2.

Also, the decellularized extracellular matrix may be derived from various tissues having fibril alignment and orientation. Desirably, the decellularized extracellular matrix may be derived from desirably one or more tissues selected from the group consisting of muscle, brain, spinal cord, tongue, airway, skin, lymph, lung, heart, liver, stomach, kidney, pancreas, intestine, skeletal muscle, fat, uterus, thymus, esophagus, salivary gland, bone, bladder, blood vessel, tendon, pancreas, thyroid gland, ligament and cartilage. More desirably, the decellularized extracellular matrix may be derived from muscle or brain tissue, but is not limited thereto. Accordingly, the finally fabricated tissue gel for transplantation may be a muscle tissue gel for transplantation or a brain tissue gel for transplantation.

Optionally, the process (a) may further include loading induced skeletal myogenic progenitor cells or induced neuronal cells into the composition. By loading the iMPCs or induced neuronal cells into the composition, the differentiation, maturation and regenerative therapeutic efficacy can be improved.

Then, the method of preparing a tissue gel for transplantation according to the present disclosure includes the process of maintaining a tensile state by applying a tensile force to a stretching device made of an elastic polymer material [process (b)].

The stretching device may be made of an elastic polymer material, specifically, an elastic material including polydimethylsiloxane (PDMS). A tensile force may be applied using elasticity thereof, and the tensile force may act in the other direction (perpendicular to the one direction) of the decellularized extracellular matrix. Specifically, a uniaxial strain of 0.1 to 1, desirably 0.3 to 0.8, may be applied from L to L + ΔL along the direction perpendicular to the one direction.

Further, the method of preparing a tissue gel for transplantation according to the present disclosure includes the process of injecting the composition prepared in the process (a) into the stretching device maintained in the tensile state in the process (b), allowing the composition to crosslink, and then removing the tensile force to align fibrils in the decellularized extracellular matrix in one direction [process (c)].

The crosslinking may be performed at a temperature of 20°C to 40°C for 15 minutes to 1 hour to achieve sufficient gelation. Also, through the removal of the tensile force, the fibrils in the decellularized extracellular matrix can be aligned in the one direction.

The method of preparing a tissue gel for transplantation according to the present disclosure may further include a process of culturing the loaded induced skeletal myogenic progenitor cells or induced neuronal cells after the tensile force is removed in the process (c) [process (d)].

The culture may be performed within 21 days at a temperature of 30°C to 40°C. Since the culture can be performed along the fibrils aligned in the one direction in the decellularized extracellular matrix, the degree of differentiation and maturation of the iMPCs or induced neuronal cells can be increased and the functionality and regenerative efficacy as skeletal muscle cells or neurons can be maximized. This phenomenon can be attributed to activation of differentiation-related signaling, activation of mechanotransduction signaling and activation of an epigenetic regulator caused by enhancement of cell-cell and cell-extracellular matrix interactions by the aligned MEM.

### Tissue gel for transplantation

The present disclosure provides a tissue gel for transplantation including a decellularized extracellular matrix which includes collagen, glycoprotein, proteoglycan, ECM-affiliated protein and ECM modulator and in which the collagen, glycoprotein, proteoglycan, ECM-affiliated protein and ECM modulator are included in an amount of 15 wt% to 45 wt%, 20 wt% to 40 wt%, 10 wt% to 20 wt%, 0.1 wt% to 10 wt% and 10 wt% to 30 wt%, respectively, with respect to a total weight of the decellularized extracellular matrix protein, and fibrils in the decellularized extracellular matrix are aligned in one direction.

The decellularized extracellular matrix is a decellularized extracellular matrix protein (particularly, decellularized skeletal muscle extracellular matrix protein), and may include collagen, glycoprotein, proteoglycan, ECM-affiliated protein and ECM modulator. The decellularized skeletal muscle extracellular matrix protein may be included in an amount of 10 wt% to 40 wt%, preferably 15 wt% to 30 wt% with respect to all of proteins in the decellularized extracellular matrix (particularly, decellularized skeletal muscle extracellular matrix). These components can form fibrils. To be specific, the collagen, glycoprotein, proteoglycan, ECM-affiliated protein and ECM modulator may be included in an amount of desirably 15 wt% to 45 wt%, 20 wt% to 40 wt%, 10 wt% to 20 wt%, 0.1 wt% to 10 wt% and 10 wt% to 30 wt%, respectively, with respect to a total weight of the decellularized extracellular matrix protein, but the present disclosure is not limited thereto. The decellularized extracellular matrix is prepared through decellularization including SDS or Triton X-100 treatment, and has a particularly high content of COL6A2, LOX, FGA, COL4A2, KERA, ITIH5, VCAN, FBN1, COL12A1 and LAMA2.

Also, the decellularized extracellular matrix may be derived from various tissues having fibril alignment and orientation. Desirably, the decellularized extracellular matrix may be derived from desirably one or more tissues selected from the group consisting of muscle, brain, spinal cord, tongue, airway, skin, lymph, lung, heart, liver, stomach, kidney, pancreas, intestine, skeletal muscle, fat, uterus, thymus, esophagus, salivary gland, bone, bladder, blood vessel, tendon, pancreas, thyroid gland, ligament and cartilage. More desirably, the decellularized extracellular matrix may be derived from muscle or brain tissue, but is not limited thereto. Accordingly, the finally fabricated tissue gel for transplantation may be a muscle tissue gel for transplantation or a brain tissue gel for transplantation.

Optionally, the tissue gel for transplantation may further include induced skeletal myogenic progenitor cells or induced neuronal cells loaded and cultured in the decellularized extracellular matrix. By loading the iMPCs or induced neuronal cells into the decellularized extracellular matrix, the differentiation, maturation and regenerative therapeutic efficacy can be improved.

Since the culture of the iMPCs or induced neuronal cells can be performed along the fibrils aligned in the one direction in the decellularized extracellular matrix, the degree of differentiation and maturation of the iMPCs or induced neuronal cells can be increased and the functionality and regenerative efficacy as skeletal muscle cells or neurons can be maximized. This phenomenon can be attributed to activation of differentiation-related signaling, activation of mechanotransduction signaling and activation of an epigenetic regulator caused by enhancement of cell-cell and cell-extracellular matrix interactions by the aligned MEM.

As a result of measuring a storage modulus of the tissue gel for transplantation, the storage modulus may be from 300 Pa to 1,000 Pa. Thus, the gelation may be successfully performed, and the tissue gel for transplantation can have excellent elastic properties.

In particular, if the artificial tissue gel fabricated according to the present disclosure is an artificial muscle tissue gel, it may be transplanted into a volumetric muscle loss (VML) animal model with a loss of 70% or more of muscle tissue and a Duchenne muscular dystrophy animal model. In this case, the artificial muscle tissue gel can have therapeutic efficacy such as great enhancement of muscle regeneration and great improvement of motor ability of the animal models.

According to the present disclosure, the tensile force is applied to the decellularized extracellular matrix in the other direction (perpendicular to the one direction) and then removed to align the fibrils in the decellularized extracellular matrix in the one direction. Thus, it is possible to effectively implement a tissue-specific microstructure. Therefore, a tissue gel for transplantation prepared according to the present disclosure can be applied not only to an artificial muscle tissue, but also to a technology for regeneration of various tissues having fibril alignment and orientation.

Also, the tissue gel for transplantation prepared according to the present disclosure can be lyophilized for a long time. Thus, it can be easily stored and can be processed into various forms such as a hydrogel and a patch. Therefore, the tissue gel for transplantation can be widely applied to tissue engineering and regenerative medicine.

In particular, a muscle tissue gel for transplantation prepared according to the present disclosure is expected to be applied as a therapeutic agent for various muscle diseases, such as traumatic muscle damage, Duchenne muscular dystrophy, muscular atrophy, and sarcopenia caused by aging, which are creating a huge market worldwide. Further, the muscle tissue gel for transplantation is expected to be highly used as an in vitro model such as a muscle disease model and a toxicity evaluation and drug screening platform.

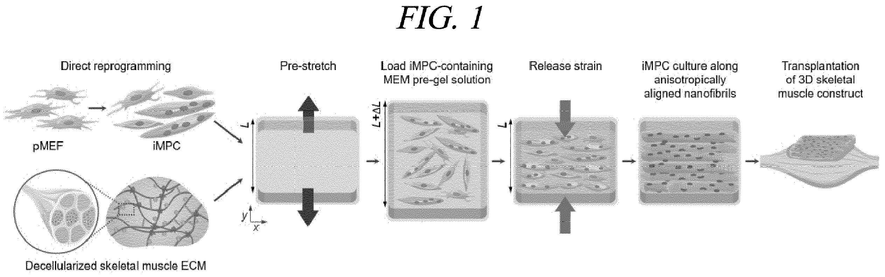

### Example 1: Fabrication of tissue-engineered artificial muscle through three-dimensional alignment of decellularized skeletal muscle extracellular matrix and induced skeletal myogenic progenitor cells (FIG. 1)

Three main elements were used to fabricate a three-dimensional skeletal muscle-like tissue.

First, a direct reprogramming technology was used to prepare induced skeletal myogenic progenitor cells (iMPCs). Herein, according to the direct reprogramming technology, genes, *MyoD, Pax7, Mef2b* and *Pitx1,* which are transcription factors important for differentiation into skeletal muscle cells, are transferred into mouse primary embryonic fibroblasts, followed by culture for about 2 weeks in myogenic induction medium to induce iMPCs forming a colony.

Second, a decellularized skeletal muscle-derived extracellular matrix (MEM) was used as a three-dimensional hydrogel scaffold.

Finally, the flexible mechanical properties of a stretching device made of polydimethylsiloxane (PDMS), an elastic polymer, and the fibrillogenesis kinetics of collagen having a fibrillary structure were used to anisotropically align fibrils of an extracellular matrix present in the MEM.

The three-dimensionally patterned MEM simultaneously provides iMPCs with a biochemical cue of the muscle tissue-specific extracellular matrix and a biophysical cue of the aligned fibrils, and, thus, it was possible to fabricate a muscle bundle-like tissue structure.

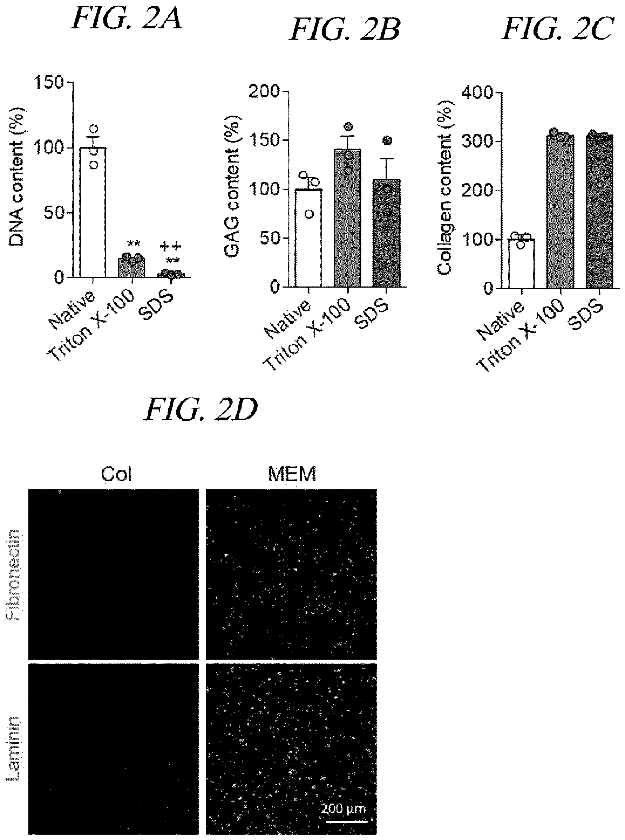

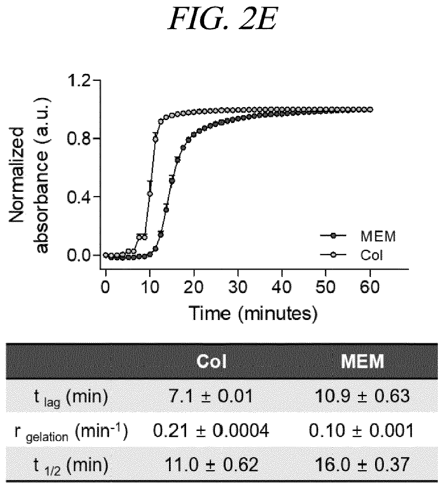

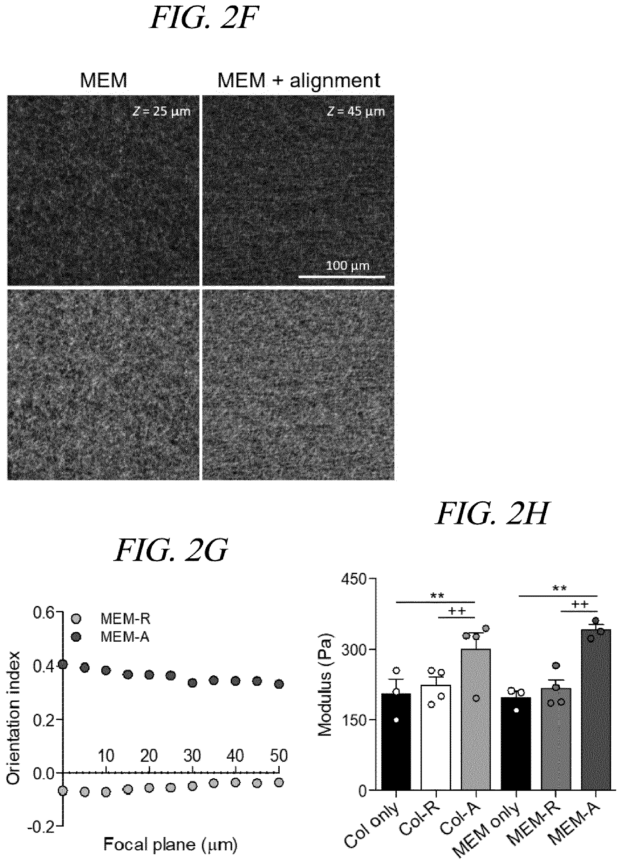

### Example 2: Analysis of decellularized skeletal muscle extracellular matrix and induction of three-dimensional anisotropic alignment of extracellular matrix (FIG. 2)

The optimization protocol for preparing a decellularized skeletal muscle extracellular matrix (MEM) is as follows: for muscle tissue, 1) treatment in distilled water for 6 to 8 hours, 2a) treatment with SDS for 48 hours or 2b) treatment with 0.2% (v/v) trypsin/ethylenediaminetetraacetic acid for 180 minutes at room temperature, followed by treatment with 0.5% (v/v) Triton X-100 for 12 hours and treatment with 1% (v/v) Triton X-100 containing 0.2% (v/v) sodium deoxycholate for 6 hours, 3) treatment in distilled water for 16 hours, 4) sterilization with 1% (v/v) penicillin/streptomycin for 2 hours (all processes performed on a shaker at 180 rpm at 4°C unless otherwise noted).
(a) As a result of quantification of DNA before (native) and after (after) decellularization of porcine skeletal muscle, it was found that more than 97% of the cells were removed compared with the native tissue.
(b) However, as a result of comparison in the amount of glycosaminoglycan (GAG), an important extracellular matrix component, it was found that there was little difference between before and after the decellularization.
(c) When the total amount of collagen was quantified by hydroxyproline analysis, the collagen (dry weight) increased by about 3 times after the decellularization.
(d) Since there is a lot of collagen in the MEM, gelation was possible through temperature-based crosslinking with the temperature adjusted to 37°C. When an MEM hydrogel (5 mg/ml) and a collagen type I hydrogel (Col, 5 mg/ml) extracted from a rat tail were stained with anti-laminin and anti-fibronectin, it was confirmed that only the MEM group was positively stained.
(e) The turbidity of the MEM and Col (5 mg/ml) was measured with a spectrophotometer to measure the absorbance thereof. Under the premise that a sample turns opaque when it becomes a gel, the turbidity of the sample was measured to observe the gelation kinetics.
(f) A hydrogel that induced fibril alignment in the MEM using the stretching device was treated with TAMRA fluorescence dye that stains collagen, and the orientation of collagen was observed through a confocal microscope (upper image). An MEM hydrogel without alignment was used as a control group. The orientation of collagen was analyzed with (g) orientation color index (lower image) and (h) orientation index through the Image J program.
(g) As a result of analyzing the orientation index at 50 micrometers inside the MEM hydrogel, it was -0.053 in the random group and about 0.33 in the align group (0 = no alignment, 1 or -1 = perfect alignment). Therefore, after a tensile force was applied, anisotropic alignment of the collagen fibrils present in the MEM was observed.
(h) The physical properties of the collagen gel and the MEM gel were measured with a rheometer under various conditions. As a result of measuring only the gels without iMPCs (Col only, MEM only), it was found that the collagen gel and the MEM gel were similar in strength (Col = 205.8 Pa, MEM = 197.6 Pa). When iMPCs were cultured without aligning fibrils, the gels showed similar strength (Col-random group = 223.2 Pa, MEM-random group = 215.5 Pa). When iMPCs were aligned, it was confirmed that the hydrogels greatly increased in strength (Col-align group= 299.9 Pa, MEM-align group = 341.5 Pa).

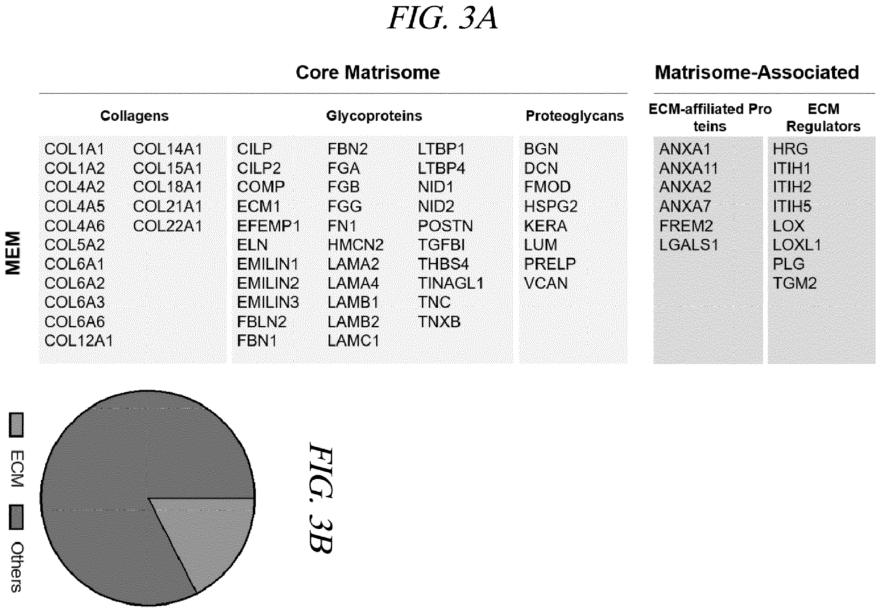

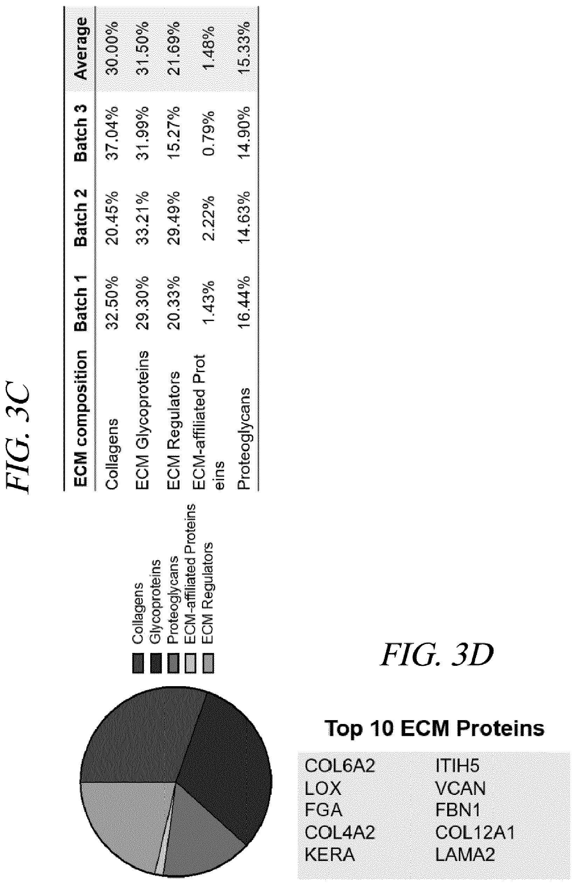

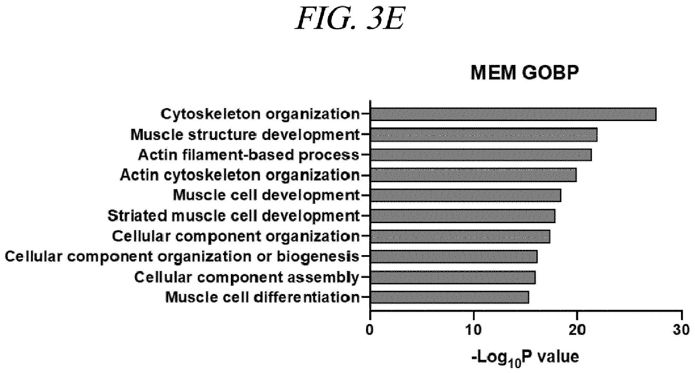

### Example 3: Proteomics of decellularized skeletal muscle extracellular matrix (FIG. 3)

The optimization protocol for preparing a decellularized skeletal muscle extracellular matrix (MEM) is as follows: for muscle tissue, 1) treatment in distilled water for 6 to 8 hours, 2) treatment with 0.2% (v/v) trypsin/ethylenediaminetetraacetic acid for 180 minutes at room temperature, followed by treatment with 0.5% (v/v) Triton X-100 for 12 hours and treatment with 1% (v/v) Triton X-100 containing 0.2% (v/v) sodium deoxycholate for 6 hours, 3) treatment in distilled water for 16 hours, 4) sterilization with 1% (v/v) penicillin/streptomycin for 2 hours (all processes performed on a shaker at 180 rpm at 4°C unless otherwise noted).
(a) As a result of proteomics to identify the components of the MEM, MEM proteins (collagen, glycoprotein, proteoglycan, ECM-affiliated protein, ECM modulator, etc.) found to be contained. Growth factor proteins such as ECM-affiliated proteins and ECM regulators are identified as including muscle-specific proteins that play an important role in muscle differentiation and development.
(b) It is found that the MEM proteins are included in an amount of 18 wt% with respect to all of proteins.
(c) The collagen, glycoprotein, proteoglycan, ECM-affiliated protein and ECM modulator are found to be included in an amount of 20.45 wt% to 37.04 wt%, 29.30 wt% to 33.2 wt%, 14.63 wt% to 16.44 wt%, 0.79 wt% to 2.22 wt% and 15.27 wt% to 29.49 wt%, respectively, in the MEM proteins.
**(d)** The 10 components that accounted for the largest portion among the MEM proteins were COL6A2, LOX, FGA, COL4A2, KERA, ITIH5, VCAN, FBN1, COL12A1 and LAMA2.
(e) The gene ontology was performed to examine the functions of proteins other than MEM proteins. As a result of analyzing the 10 GOs with the highest logarithmic p-values, it is confirmed that they play an important role in muscle differentiation, muscle construction, and muscle development.

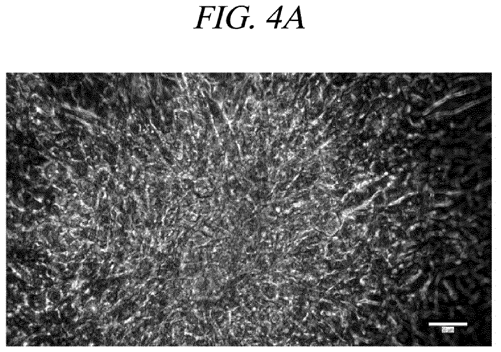

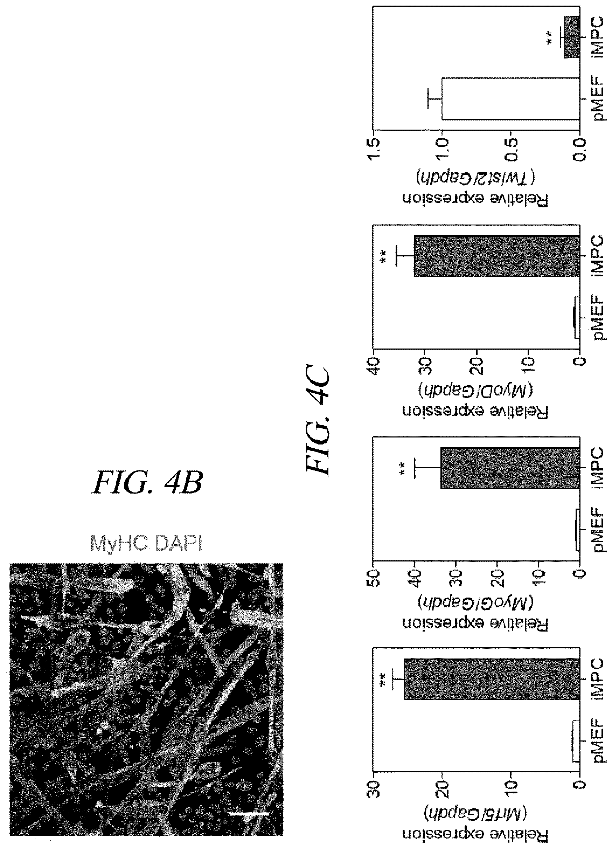

### Example 4: Analysis of induced skeletal myogenic progenitor cells produced by using direct reprogramming technology (FIG. 4)

(a) An optical microscope image of induced skeletal myogenic progenitor cells (iMPCs) on day 14, and (b) myosin heavy chain (MyHC) immunostaining data.
(c) As a result of gene analysis of the iMPCs and primary mouse embryonic fibroblasts (pMEFs) on day 14, the expression levels of myogenic factor 5 (*Mrf5*)*,* myogenin (*MyoG*) and myogenic differentiation 1 (*MyoD1*)*,* muscle-specific markers, were higher in the iMPCs than in the pMEFs, and the expression level of *Twist2,* a fibroblast marker, was lower in the iMPCs than in the pMEFs.

Therefore, it was confirmed that the iMPCs produced by transferring a transcription factor gene plasmid twice in total using each of electroporation [Neon, Thermo Fisher Scientific] and Lipofectamine 2000, a gene carrier, once have muscle-specific phenotypes.

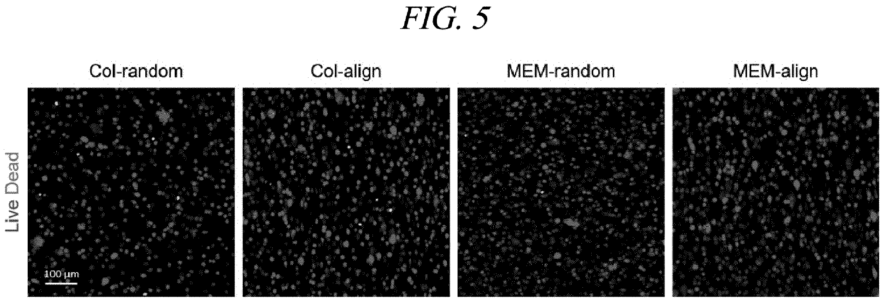

### Example 5: Analysis of viability of induced skeletal myogenic progenitor cells three-dimensionally aligned in MEM hydrogel (FIG. 5)

As a result of analyzing the viability of cells through live/dead analysis after inducing fibril alignment in the MEM, it was confirmed that there were almost no dead cells.

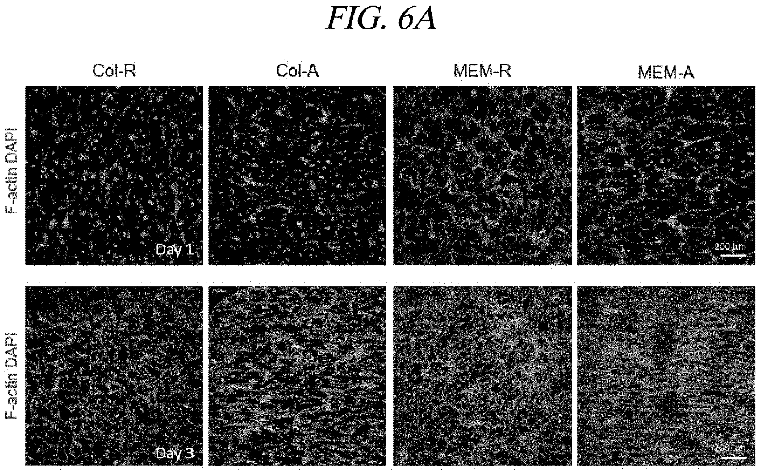

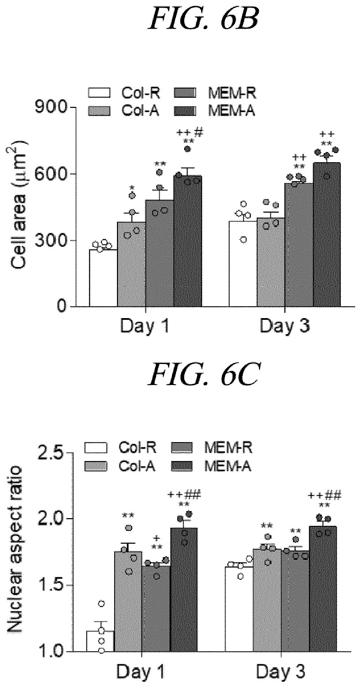

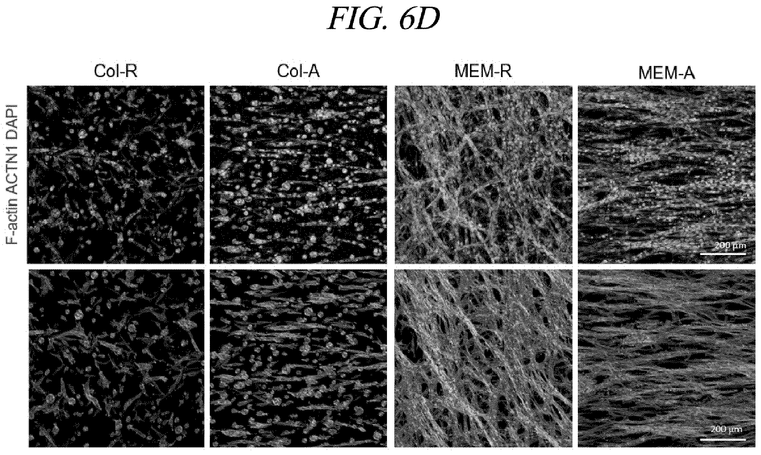

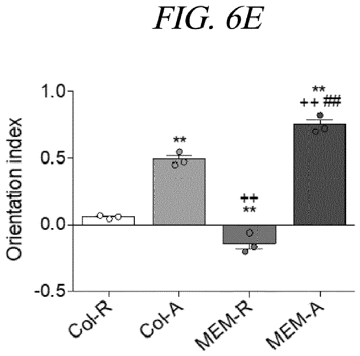

### Example 6: Analysis of iMPCs aligned in MEM hydrogel (FIG. 6)

(a) After anisotropic alignment was induced, cell alignment was confirmed by observing the morphology of the iMPCs cultured for 1 day and 3 days in a 3D MEM gel and a Col gel through filamentous-actin (F-actin) staining.
(b) When the cell area was quantified, each of the Col-align group, the MEM-random group, and the MEM-align group had a significantly greater cell area than the Col-random group on day 1 of culture, and in particular, the MEM-align group had the greatest cell area. The cell area of the Col-random group became greater on day 3 of culture than on day 1, and became similar to that of the Col-align group. Overall, the MEM groups (the MEM-random group and the MEM-align group) had greater cell areas. It was found that the MEM has a ligand important for cell adhesion and thus provides more favorable conditions for cell extension.
(c) According to a previous report, the shape of the nucleus extends during differentiation into muscle. When the nuclear aspect ratio was quantified, each of the Col-align group, the MEM-random group, and the MEM-align group had a higher nuclear aspect ratio than the Col-random group on day 1 of culture. In particular, the MEM-align group had the highest nuclear aspect ratio. The nuclear aspect ratio of the Col-random group became higher on day 3 than on day 1, and became similar to those of the Col-align group and the MEM-random group. Even on day 3, the MEM-align group had the highest nuclear aspect ratio.
(d, e) When the orientation index (Ol) was quantified on day 5 of culture, the align groups, i.e., the Col-align group and the MEM-align group, had orientation indexes of 0.49 and 0.75, respectively. However, the random groups had indexes close to 0.
   - Watanabe, T. M. et al. Chromatin plasticity as a differentiation index during muscle differentiation of C2C12 myoblasts. Biochem. Biophys. Res. Commun. 418, 742-747 (2012)
   - Hwang, Y. et al. Directed in vitro myogenesis of human embryonic stem cells and their in vivo engraftment. PIoS one 8, e72023-e72023 (2013).

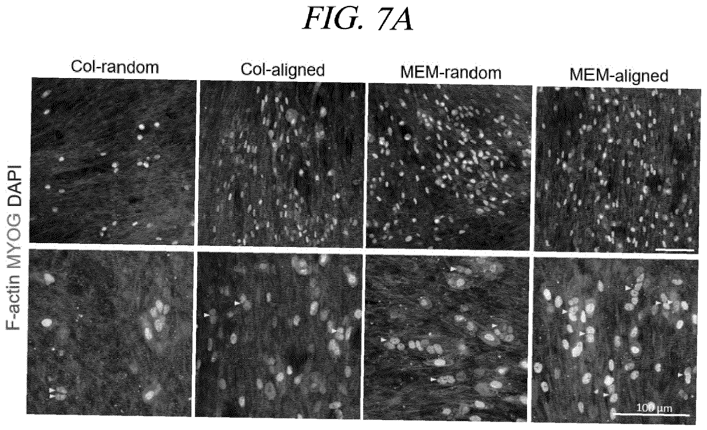

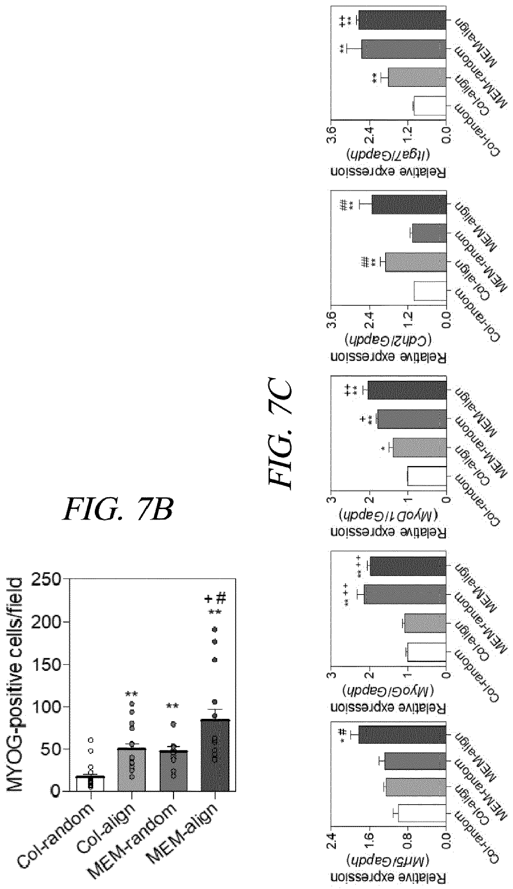

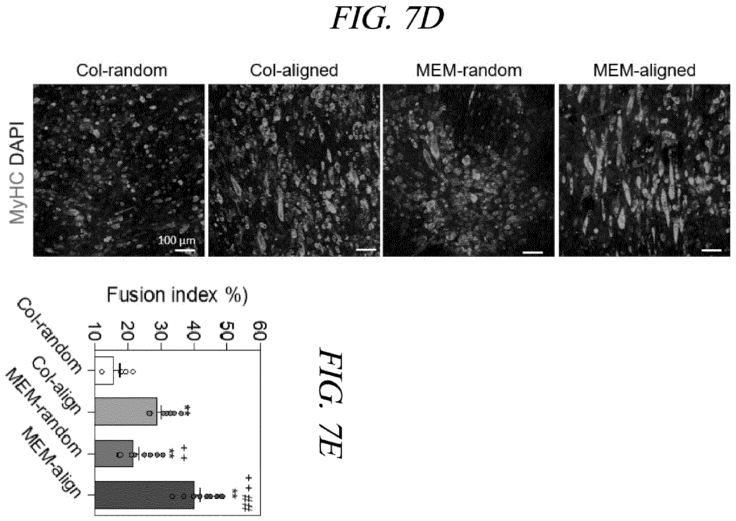

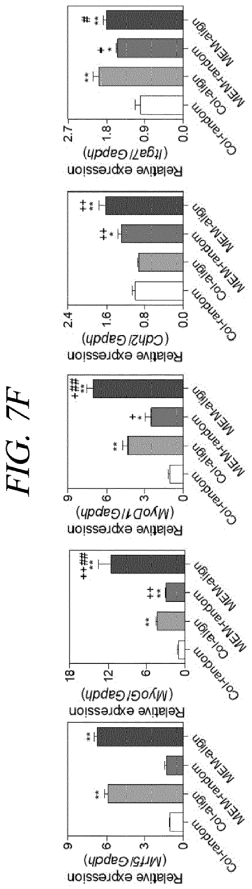

### Example 7: Analysis and assessment of degree of differentiation of iMPCs aligned in 3D MEM hydrogel (FIG. 7)

(a) As a result of immunostaining of filamentous actin (F-actin) and Myogenin (MYOG), a muscle-specific marker, on day 5 of culture, fused MYOG-positive multinucleated cells began to appear.
(b) As a result of quantitative analysis of the MYOG-positive cells, the MEM-align group showed the highest number of the MYOG-positive cells.
(c) On day 5 of culture, the expression levels of genes related to muscle differentiation were analyzed by quantitative PCR (qPCR). As for Mrf5, it was found that there was no difference in expression level among the Col-random group, the Col-align group, and the MEM-random group, but the expression level increased only in the MEM-align group. As for MyoG, the expression level was about two times higher in the MEM groups than in the Col groups. As for MyoD1, the expression level was increased in the three groups compared with the Col-random group. The gene expression level was higher in the MEM-random group than in the Col-align group and higher in the MEM-align group than in the MEM-random group. Cdh2 is known to play an important role in skeletal muscle differentiation as a calcium-dependent cell-cell adhesion factor, and the expression level was significantly high in the Col-align and MEM-align groups. The expression level of integrin alpha (Itga7), which is known to be highly expressed in skeletal muscle, was increased in the three groups compared with the Col-random group. In particular, the MEM-random group and the MEM-align group showed the highest expression levels. Overall, it was found that the expression of myogenic markers was increased in the MEM groups, and the expression level of Cdh2, a cell-cell adhesion marker, was high in the align groups.
(d) Myosin heavy chain (MyHC) immunostaining was performed on day 10 of culture.
(e) As a result of quantifying the degree of cell fusion by the fusion index, the Col-random group, the Col-align group, the MEM-random and the MEM-align groups had fusion indexes of 15.8 ± 2.01%, 28.8 ± 1.30%, 21.8 ± 1.74% and 40.2 ± 1.81%, respectively. Physical alignment of cells may increase the likelihood that fusion will occur, and an ECM component in the MEM may promote cell fusion. For example, laminin served to align prior to fusion during myogenic differentiation.
(f) On day 10 of culture, a qPCR analysis was performed. The gene expression levels of *Mrf5, MyoG, MyoD1* and *Itga7* were lower increased in the three groups compared with the Col-random group. In particular, the gene expression level was significantly high in the align groups, and the MEM-align group showed the highest expression level. As for Cdh2, the gene expression level was high in the MEM groups. On day 10, an alignment factor seems to be a more important factor in inducing cell fusion and enhancing myogenic marker expression than a biochemical component. In summary, these results indicated that both physical signals induced by cell alignment and biochemical signals provided by the MEM promoted myogenic differentiation of the iMPCs.

Overall, it seems that the effect of the MEM is greater at the initial stage of differentiation of the iMPCs (to day 5), and as the differentiation progresses (to day 10), the effect of the cell alignment is greater.
- Sanes, J. R. The basement membrane/basal lamina of skeletal muscle. J. Biol. Chem. 278, 12601-12604 (2003)
- Mayer, U. Integrins: redundant or important players in skeletal muscle J. Biol. Chem. 278, 14587-14590 (2003).

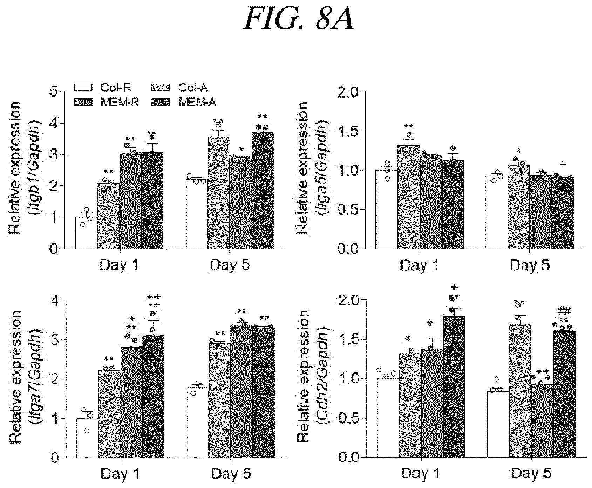

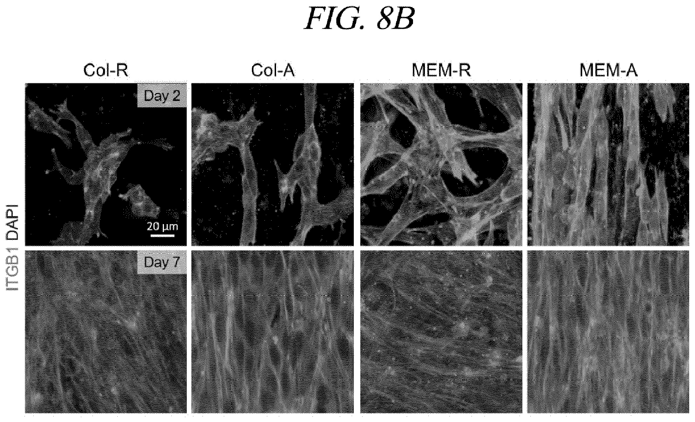

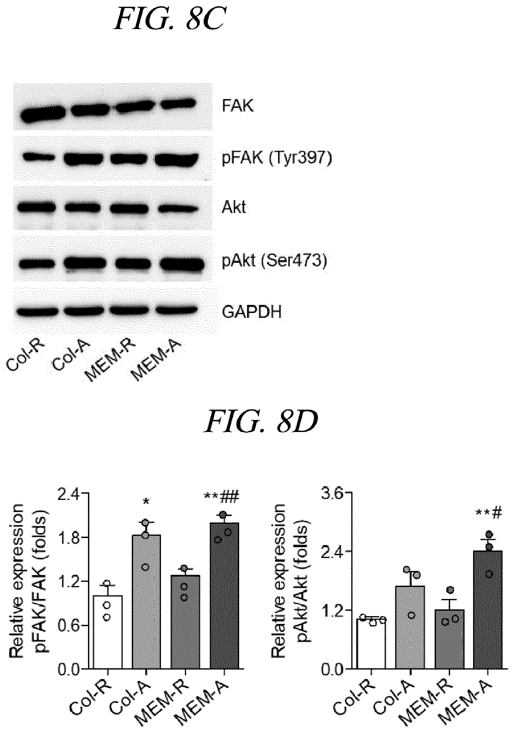

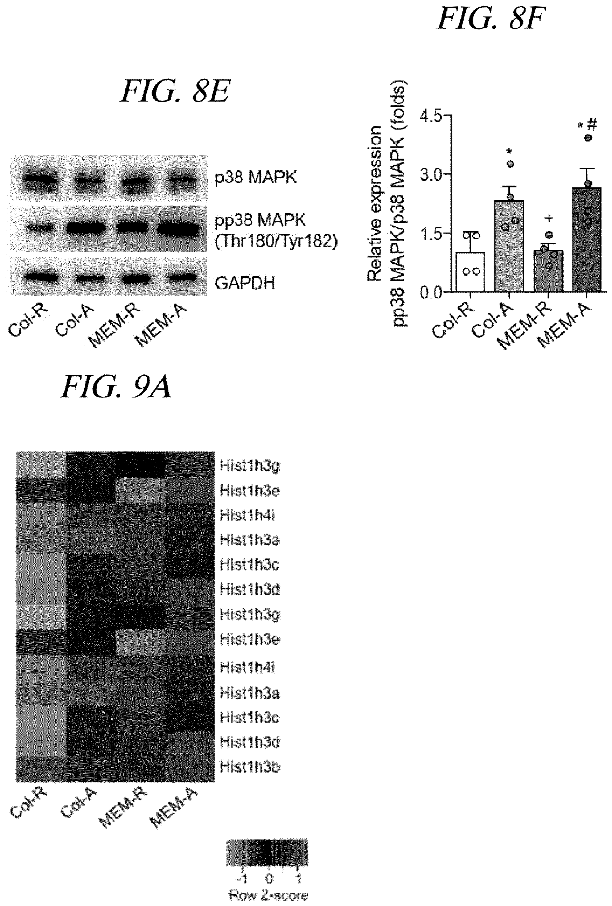

### Example 8: Analysis of differentiation-related signaling mechanism of iMPCs cultured in 3D MEM hydrogel (FIG. 8)

(a) Integrin acts as a receptor for many ECM ligands and is a transmembrane heterodimer composed of α- and β-chains connected to actin cytoskeleton. Integrin expression varies during myogenesis. In particular, it is known that β1 subunit integrin is most closely related to integrin that binds to α1, α3, α4, α5, α6, α7, α9 and αv subunit. Specifically, α5β1 integrin is a receptor for fibronectin, and α6β1 and α7β1 are important functional groups that bind to laminin and play an important role in stabilizing the muscle during myogenesis. Also, α5β1 and α6β1 are broadly expressed and downregulated after myotube formation, whereas α7β1 is limited to mainly the skeletal and myocardium and strongly upregulated during myofiber fusion.

The expression integrin beta1 (Itgb1), integrin alpha5 (Itga5) and -7 (Itga7) was analyzed by qPCR on day 1 and day 5 of culture. On day 1, the expression level of Itgb1 was increased in the three groups compared with the Col-random group. In particular, the MEM-random group and the MEM-align group showed the highest increases by about 3 times. On day 5, the Col-align group and the MEM-align group showed the highest expression levels. Specifically, the expression level of ltga5 on day 1 of culture was similar to that on day 5. Also, the expression level of Itga7 was increased in the three groups on day 1 and day 5 compared with the Col-random group. In particular, the MEM-random group and the MEM-align group showed the highest expression levels. On day 5, the expression level of Itga7 increased to be similar among the Col-align, MEM-random and MEM-align groups. The expression levels of Itgb1 and Itga7 greatly increased in the MEM groups on day 1, but increased in the Col groups on day 5. The expression level of ltga5 did not differ significantly among the groups, so it is presumed that it does not play an important role in culturing the iMPCs in the MEM hydrogel.

On day 1, the expression level of Cdh2 was about 1.3 times higher in the Col-align group and the MEM-random group than in the Col-random group and about 1.8 times higher in the MEM-align group than in the Col-random group. The expression level of Cdh2 in the Col-align group on day 5 was about 1.7 times higher than that in the Col-random group on day 1, and became similar to that in the MEM-align group. The MEM-random group showed a similar expression level to the Col-random group. Therefore, it was found that cell alignment increased the expression of Cdh2 on day 5. Cdh2 is known to affect cell-cell adhesion and actin assembly of myoblasts and thus affect cell fusion and differentiation.

(b) As a result of immunostaining of integrin β1 on day 2 and day 7, the MEM groups showed high expression levels on day 2. On day 7, the expression level was higher in the MEM-random group, the Col-align group and the MEM group than in the Col-random group.

(c, d) Protein expression levels of focal adhesion kinase (FAK) and protein kinase B (Akt) were analyzed by western blot.

Referring to the expression levels of total FAK and phosphorylated FAK [pFAK(tyr397)], there was no difference for total FAK, but the expression level of pFAK(tyr397) was increased in all the three groups compared with the Col-random group. In particular, the align groups showed the highest increases (MEM-random group: 1.43 ± 0.08 times, Col-align group: 1.89 ± 0.22 times, and MEM-align group: 2.09 ± 0.10 times).

Akt also showed a similar result. The expression of total Akt was similar among the groups, but the expression level of pAkt(ser473) increased by about 1.5 times in the align groups (Col-align group: 1.54 ± 0.14 times, MEM-random group: 1.13 ± 0.10 times, MEM-align group: 1.66 ± 0.02 times). It was found from the present results that FAK and Akt signaling are stimulated by cell alignment and the MEM, and that particularly the cell alignment has a greater effect on activation of FAK and Akt. Therefore, it was found that when cell alignment is induced, the expression of integrin increases and integrin binding increases, and, thus, FAK phosphorylation increases.

(e, f) MAPK is known to be activated by E-cadherin and E-cadherin binding. The expression of the phosphorylated MAPK increased in the align groups (the Col-A group and the MEM-A group), which verified that the induction of cell alignment increased cell-cell interaction.

That is, it was confirmed that the differentiation of the iMPCs can be enhanced by inducing integrin expression and binding, cell-cell adhesion, and muscle differentiation-related signaling (FAK, AKT, MAPK) through the MEM and cell alignment.
- Redfield, A., Nieman, M. T. & Knudsen, K. A. Cadherins promote skeletal muscle differentiation in three-dimensional cultures. J. Cell Biol. 138, 1323-1331 (1997)
- Charrasse, S. et al. N-cadherin-dependent cell-cell contact regulates Rho GTPases and betacatenin localization in mouse C2C12 myoblasts. J. Cell Biol. 158, 953-965 (2002).

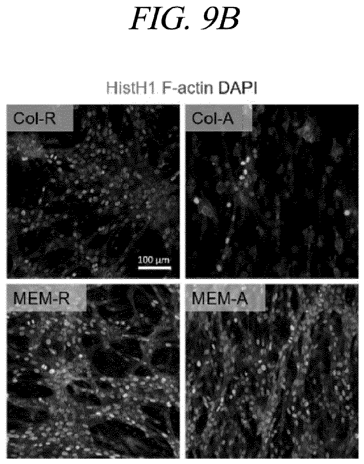

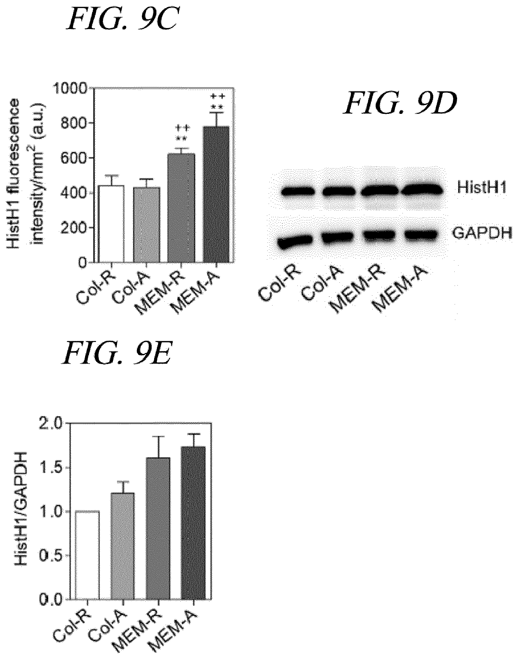

### Example 9: Analysis of epigenetic regulators in iMPCs cultured in 3D MEM hydrogel (FIG. 9)

(a) It was confirmed that Histone 1 gene expression increased in iMPCs cultured for 3 days in a 3D MEM hydrogel. Specifically, Histone 1 gene expression in the iMPCs cultured in the 3D MEM hydrogel under various conditions was analyzed by RNA-sequencing analysis. When the differences in expression were presented in a heat-map, it was confirmed that several Histone 1 gene variants were highly expressed in the MEM groups (particularly, the MEM-A group).
(b-c) From the result of Histone H1 immunostaining, it is observed that the protein expression level of Histone H1 is increased in the MEM groups (particularly, the MEM-A group).
(d-e) Western blot analysis showed a similar tendency thereto.

This result suggests that muscle-specific extracellular matrix components can enhance muscle differentiation through epigenetic regulators in the iMPCs.

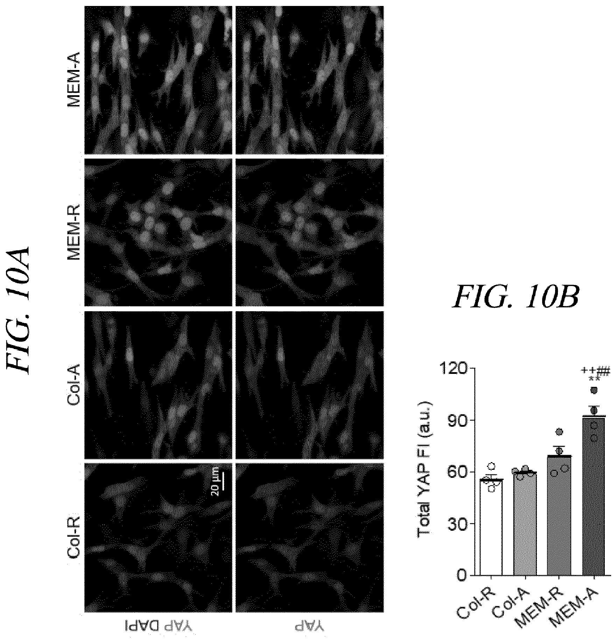

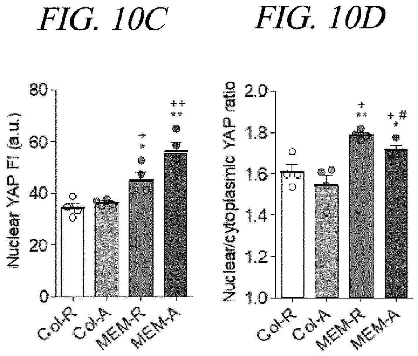

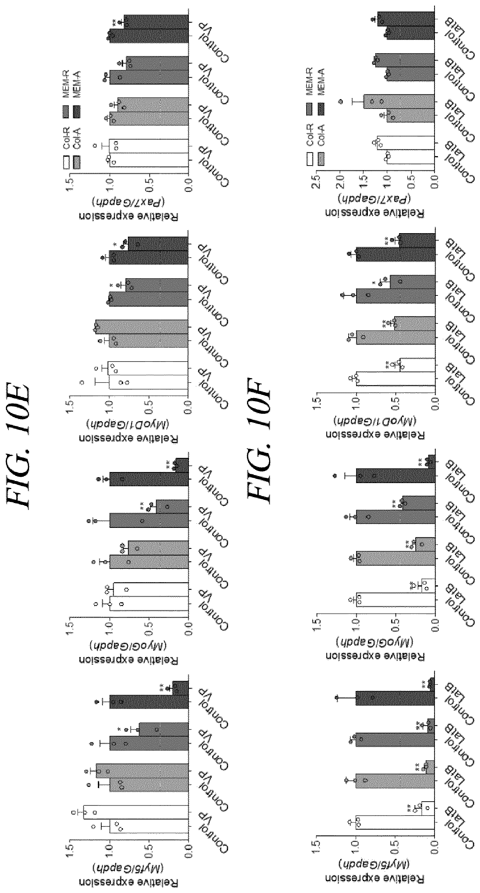

### Example 10: Analysis of mechanotransduction signaling in iMPCs cultured in 3D MEM hydrogel (FIG. 10)

(a-d) The expression of YAP, a mechanotransduction signaling regulator, in the iMPCs was quantified by immunostaining on day 3 after cell alignment. When a total fluorescence intensity of YAP was quantified, the intensity was significantly higher in the MEM groups than in the Col groups. In particularly, the MEM-align group showed the highest intensity (Col-random group: 34.3 ± 1.68, Col-align group: 36.3 ± 1.24, MEM random group: 45.1 ± 6.04, MEM align group: 56.3 ± 7.15). When the nuclear:cytoplasmic ratio of YAP fluorescence intensity was quantified, the Col-random group, the Col-align group, the MEM-random group, and the MEM-align group showed the ratios of 1.56 ± 0.02, 1.55 ± 0.07, 1.79 ± 0.01, and 1.69 ± 0.01, respectively. The ratio was generally higher in the MEM groups than in the Col groups. Therefore, it was found that activated YAP (YAP present in the nucleus) was present in a greater amount in the MEM groups.
(e, f) To analyze the role of YAP in the 3D MEM, verteporfin and latrunculin B, YAP inhibitors, were added to the cell culture medium and cultured for 3 days. Verteporfin (VP) inhibited the function of YAP by disrupting a YAP-transcriptional enhanced associate domain (TEAD) complex. As for Pax7, verteporfin did not affect the expression level of Pax7 mRNA in any group. As for Myf5, MyoD1 and MyoG, the Col groups did not show a significant difference in expression after VP treatment, but a remarkable decrease in expression level was observed in the MEM groups at the time of VP treatment. Therefore, it was found that when the function of YAP was inhibited, muscle differentiation was inhibited only in the MEM groups. Latrunculin B (LB) inhibited the polymerization of F-actin monomers and thus allowed YAP/TAZ factors to remain in the cytoplasm. YAP/TAZ could not be activated because they could not migrate into the nucleus. As a result of LB treatment, the gene expression of Mrf5, MyoD1 and MyoG decreased in all the groups, but Pax7 was not significantly affected. F-actin plays an important role in myogenesis, and since LB inhibited F-actin polymerization, very low expression levels of muscle-specific markers were observed in all the groups.

As a result, it is inferred that the 3D MEM environment increases the expression and activation of YAP and thus increases the myogenesis of the iMPCs through the activation of YAP. This was confirmed by the fact that when YAP signaling was inhibited through treatment with the YAP inhibitors such as VP and LB, differentiation was more significantly affected and thus decreased in the MEM groups.

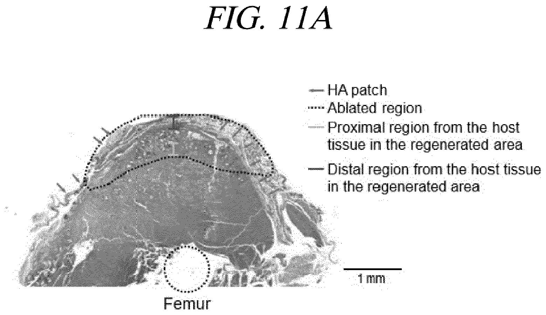

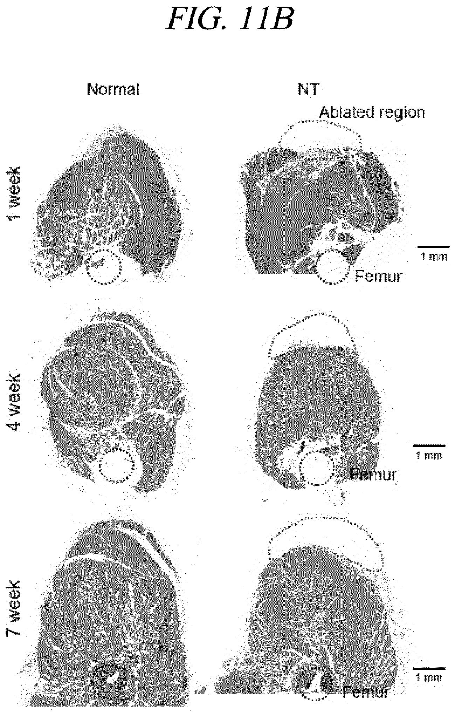

### Example 11: Fabrication of volumetric muscle loss (VML) mouse model (FIG. 11)

As an animal model for severe muscle damage caused by severe trauma, tumor resection or infection, a VML model was prepared by ablating 75% of the quadriceps femoris (QF) muscle area of a mouse (90% of the fascia latae and 60% of the rectus femoris) with a surgical knife. An MEM-iMPC muscle structure [size: 3 mm (x) ×5 mm (y) ×1 mm (z)] was placed on the ablated region, and a hyaluronic acid catechol conjugate patch [size: 7 mm (x) × 1 mm (y) × 2 mm (z)] covered over the structure to fix it. Gelation was induced by oxidizing the patch with 4.5 mg/mL NalO₄. After 2 to 3 minutes, it was washed with PBS, and transplantation was completed.
(a) Referring to the cross-sectional image after hematoxylin and eosin (H&E) staining at week 1 of transplantation of the MEM-A muscle structure into the ablated region, the black dots indicate the muscle-ablated region and the yellow-green arrows indicate the remaining patch. When a pathological analysis was performed at weeks 1, 4 and 7, a proximal region with respect to the host muscle tissue was marked in yellow and a distal region with respect to the host muscle tissue was marked in red.
(b) A histological analysis was performed at weeks 1, 4 and 7 after transplantation, and it was confirmed that no muscle was regenerated in a group without treatment (NT).
   - Shin et al., Tissue Tapes-Phenolic Hyaluronic Acid Hydrogel Patches for Off-the-Shelf Therapy (2019)

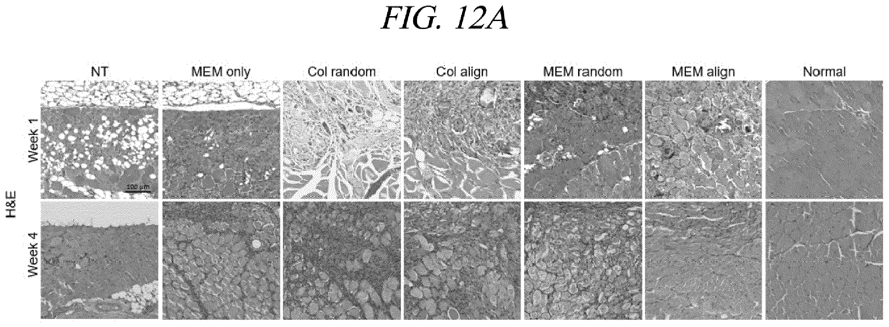

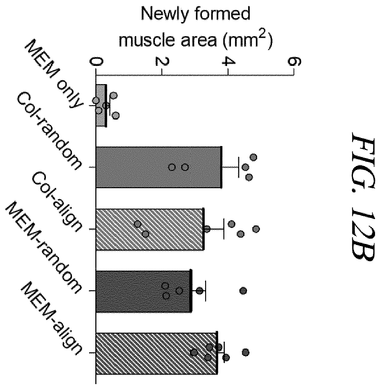

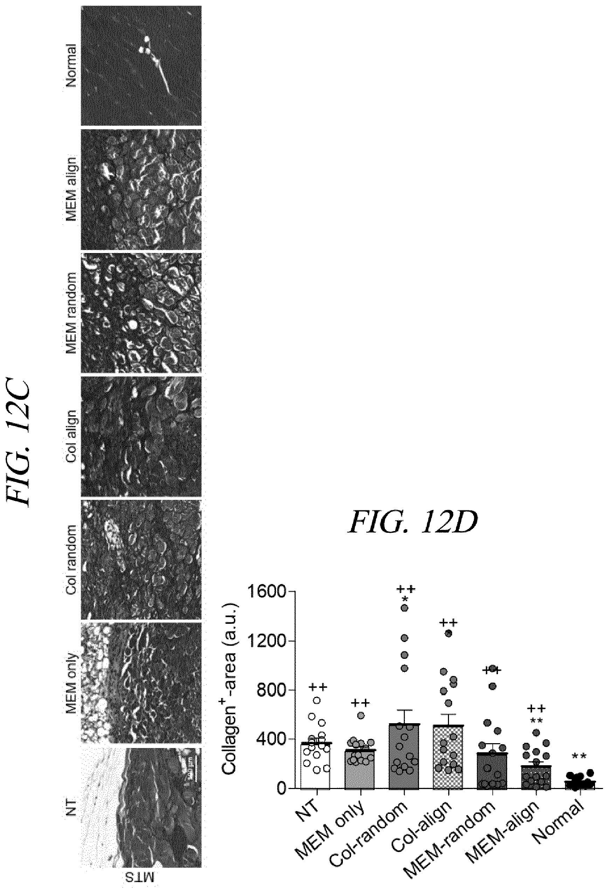

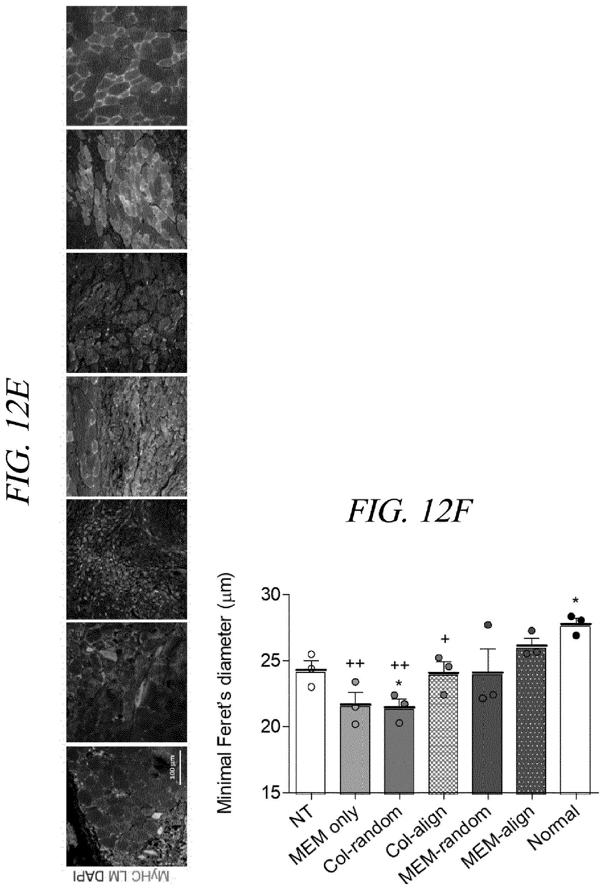

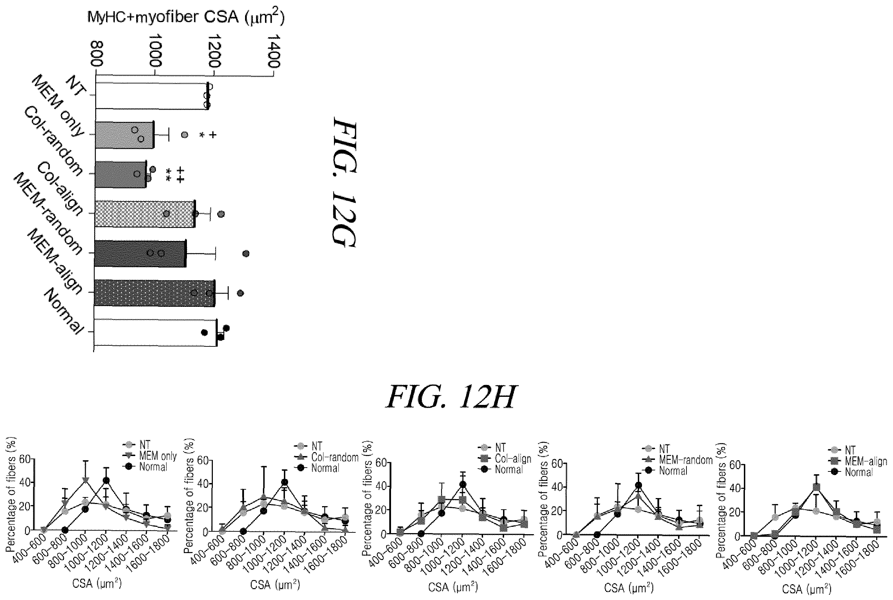

### Example 12: Histological analysis (weeks 1 and 4) after transplantation of artificial muscle tissue into volumetric muscle loss (VML) mouse model (FIG. 12)

(a) At weeks 1 and 4 after transplantation, no newly generated muscle was observed in the no treatment group (NT) and a group transplanted with an MEM hydrogel only (MEM only). Also, at week 1, fat deposition and small dystrophic myofibers were observed in the NT and M EM only groups. In all groups (Col-random, Col-align, MEM-random and MEM-align groups) transplanted with iMPCs, it was confirmed that the transplanted structure was well integrated with the host tissue. As a result of analyzing the newly generated tissue, a lot of non-myogenic fibers were still observed in the Col groups. In the MEM groups, small myotubes and large plump mature dystropic myofibers with the nucleus in the center were observed to be mixed, which indicated that the muscle was being regenerated. At week 4, in the NT and MEM only groups, cellular infiltration was observed in the vicinity of the muscle-ablated region and regenerative fibers with the nucleus in the center were also observed. However, no newly generated muscle mass was observed.
(b) As a result of quantifying the newly generated tissue mass at week 4, the newly generated muscle tissue increased dramatically only in the group transplanted with the iMPCs.
(c) Masson's trichrome (MT) staining images at week 4 of transplantation
(d) The degree of fibrosis was quantitatively analyzed by MT staining. Fibrotic collagen deposition occurred in all the groups compared with the Normal group. Among all the groups, the MEM-align group showed the smallest collagen deposition area at a level most similar to that of the Normal group, which confirmed that the lowest degree of fibrosis occurred in the MEM-align group.
(e) As a result of immunostaining with MyHC and laminin, MyHC-positive myofibers were observed in the four groups transplanted with the iMPCs. However, correct laminin deposition was observed only in the Col-align, MEM-random and MEM-align groups. A large number of non-myogenic cells were seen in the newly generated tissue of the Col-random, Col-align and MEM-random groups. Meanwhile, in the MEM-align group, newly generated myofibers were widely distributed.
(f) Then, the size of myofibers was quantified. When the minimum Feret diameter was quantified, all the groups except the MEM-align group (the NT, MEM only, Col-random, Col-align and MEM-random groups) showed a lower diameter than the Normal group. The MEM-align group showed a similar diameter to the Normal group. Therefore, it was found that only in the mice transplanted with MEM-align tissue structure, muscles were generated with a similar size to normal muscles.
(g) When myofiber cross-sectional area (CSA) was quantified, the M EM only and Col-random groups showed significantly small areas, and the NT, Col-align, MEM-random and MEM-align groups showed similar areas to the normal mouse.
(h) When myofiber frequency distribution was analyzed, it was found that the heterogeneity of myofiber areas increased in the NT groups compared with the normal group, and the distribution pattern depending on the size was very different. The MEM-only and MEM-random groups showed more similar CSA distribution patterns to the Normal group than to the NT group. However, the graph curve was skewed to the left, which indicated that the degree of distribution of myofibers with a small CSA was higher than that of the Normal group. The col-random and Col-align groups showed more similar patterns to the NT group than to the Normal group, and also showed a high degree of distribution of small-sized myofibers. Among all the groups, the CSA distribution frequency of the MEM-align group was the most similar to that of the Normal group. Therefore, it was confirmed that the newly generated myofibers in the MEM-align group expressed the most mature phenotype.

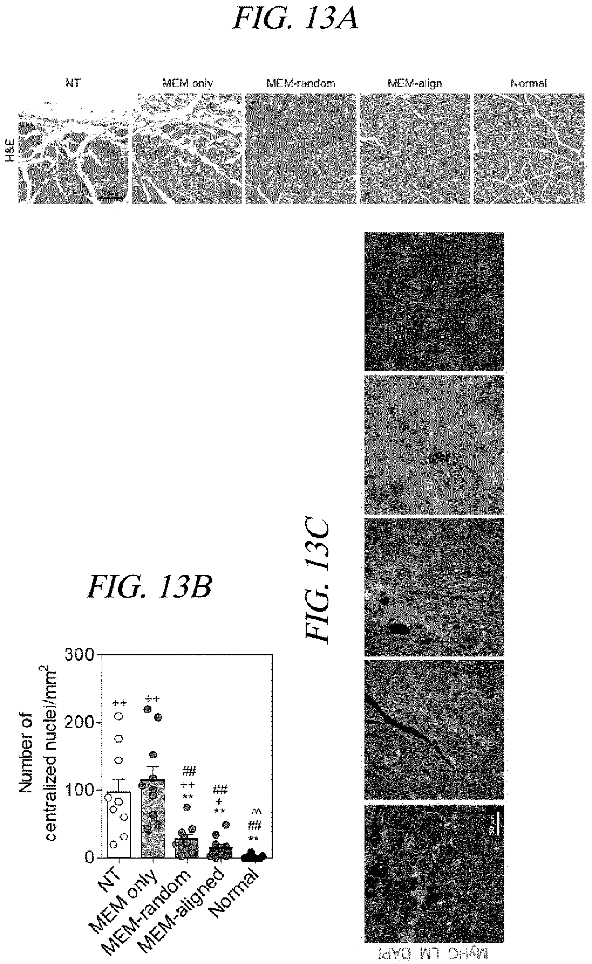

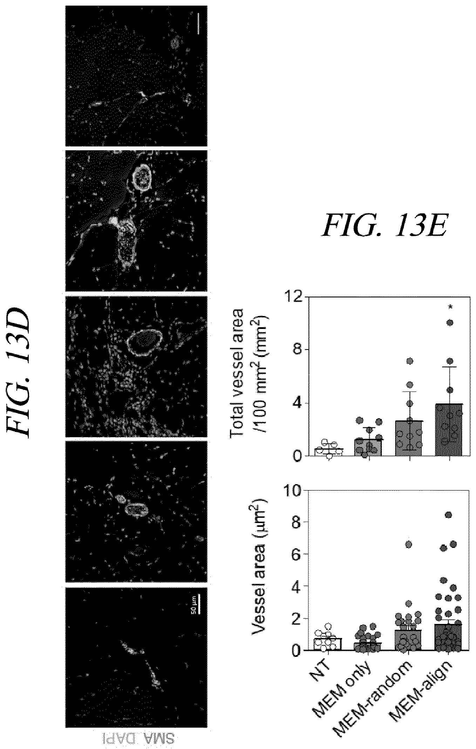

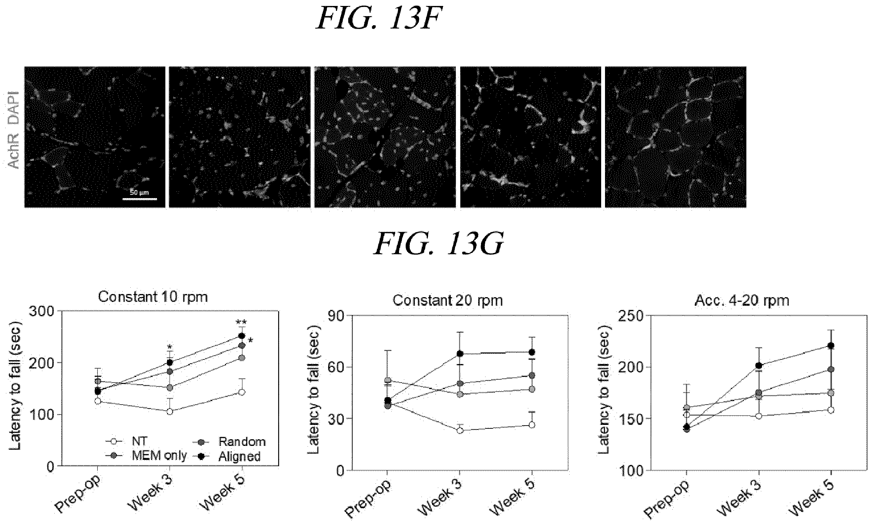

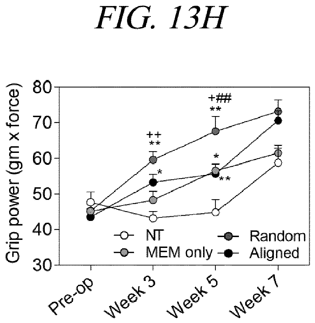

### Example 13: Histological analysis (week 7) and motor ability/ behavior assessment after transplantation of artificial muscle tissue into volumetric muscle loss (VML) mouse model (FIG. 13)

(a, c) H&E staining and immunostaining were performed to the MEM-only, MEM-random and MEM-align groups at week 7 of transplantation. The shape of the newly regenerated myofibers in the MEM-align group showed the most mature phenotype. In the MEM-random group, non-muscle cells not stained with MyHC were still seen between myofibers. In the NT and MEM-only groups, excessive connective tissue and myofibers being regenerated were observed from the damaged tissue.
(b) When cells with the nucleus in the center were quantified, a large number of cells were observed particularly in the NT and MEM-only groups, which indicated that a larger number of early stage immature myofibers were present in these two groups. However, the MEM-random and MEM-align groups contained a larger number of mature myofibers.
(d, e) It was confirmed that the SMA-positive microvessel area and the number of vessels were significantly higher in the MEM-random and MEM-align groups than in the NT and MEM-only groups.
(f) In addition to vascularization, muscle innervation is important for muscle function. Damage to muscle innervation caused by muscle damage can lead to muscle atrophy and loss of function. Therefore, the formation of a neuromuscular junction containing a mature acetylcholine receptor (AchR) is important for functional regeneration of myofibers, and, thus, after treatment with the MEM-align tissue structure fabricated according to the present disclosure, the presence of AchR in the newly regenerated tissue was checked. The expression level of AchR in the MEM-random and MEM-align groups was higher than that in the NT and MEM only groups, and was similar to that in the Normal group.
(g) In order to test motor neuron function and skeletal muscle function, a rotarod test and a hindlimb grip strength test were performed.

Rotarod test: When the muscle endurance of a mouse was assessed by placing the mouse on a belt wound on a cylinder and rotating the cylinder at a constant speed or with an acceleration, the mouse of the MEM-align group ran significantly longer than the mouse of the NT group at a speed of 10 rounds per minute (rpm). It was confirmed that the most inspired functional skeletal muscle formation was achieved in the mouse of the MEM-align group (week 3; NT group: 105.8 ± 108.0, MEM only group: 151.5 ± 122.7, MEM-random group: 182.9 ± 116.5, MEM-align group: 200.7 ± 94.2, Week 5; NT group: 143.1 ± 112.0, MEM only group: 209.6 ± 113.5, MEM-random group: 233.6 ± 98.5, MEM-align group: 252.4 ± 72.4). Although it was not statistically significant, a similar tendency observed at a speed of 20 rpm and with an acceleration of from 4 rpm to 20 rpm verified that the greatest improvement in muscle endurance was achieved in the MEM-align group.

Hindlimb grip strength test: All the treatment groups showed a higher grip strength than the NT group. The MEM-align group showed significantly higher values than the MEM-only group at week 3 and week 5, and also showed a significantly higher value than the MEM-random group at week 5. This serves as motor ability assessment data showing that the bioengineered skeletal muscle structure fabricated by the combination of a decellularized extracellular matrix and three-dimensional alignment of iMPCs has great potential as a therapeutic strategy to treat VML.

That is, when the artificial muscle tissue prepared with the 3D MEM-align and the iMPCs was transplanted, the regeneration of new muscle tissue was greatly enhanced and the functional recovery of damaged muscle was promoted, which verified that the artificial muscle tissue induced enhancement of muscle hypertrophy, muscle strength, and muscular endurance important for muscle regeneration and development.

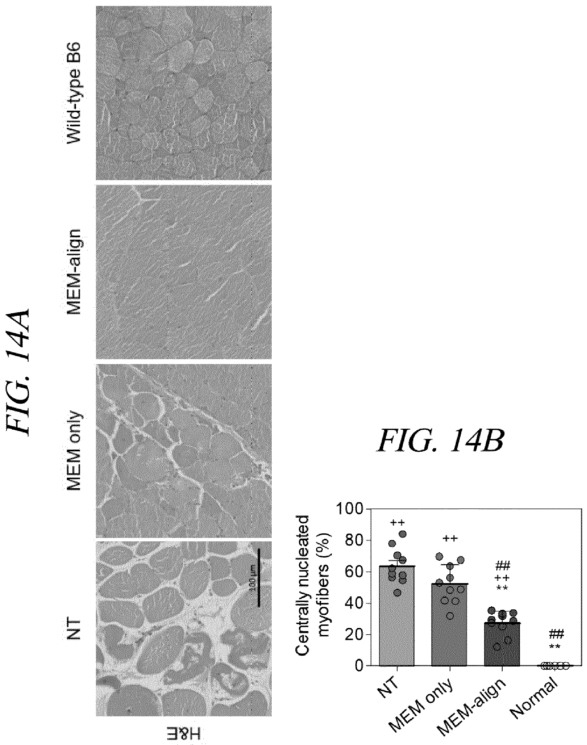

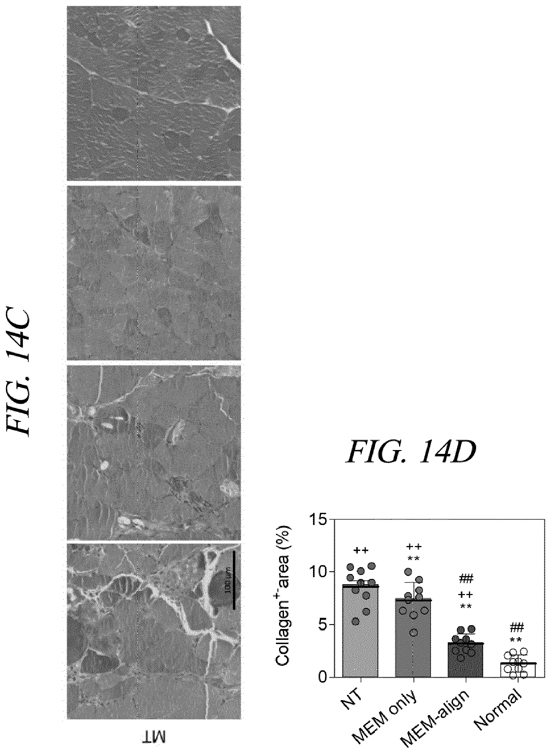

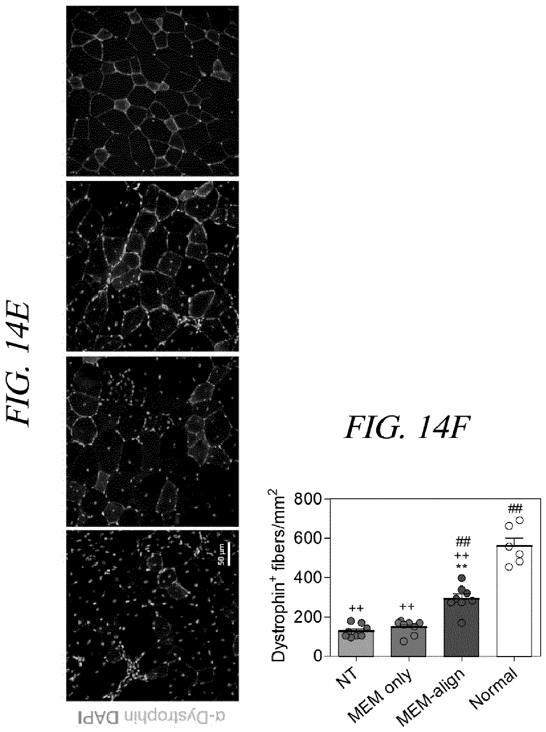

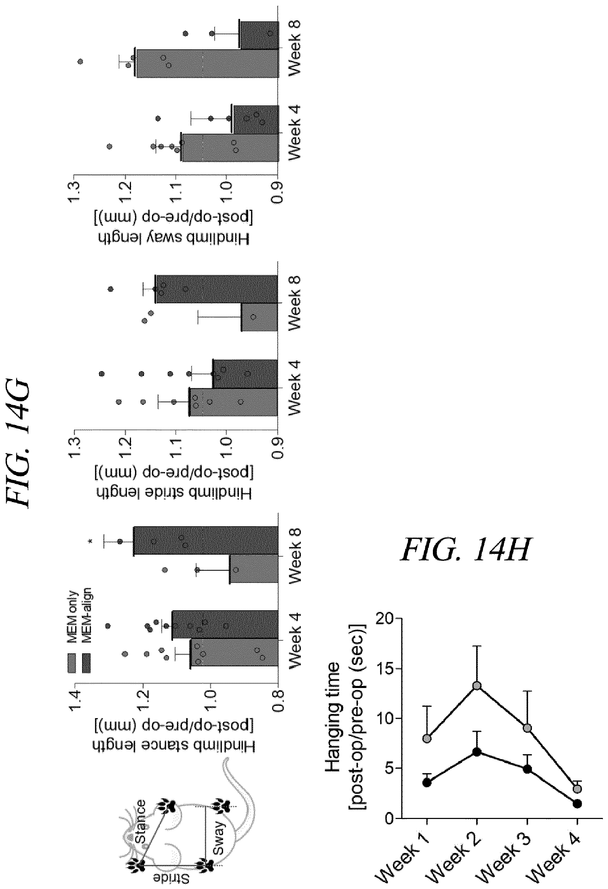

### Example 14: Histological analysis and kinematic analysis and assessment after transplantation of artificial muscle tissue into MDX transgenic mouse model of Duchenne muscular dystrophy (DMD) (FIG. 14)

(a, b) To assess the applicability of an MEM-align tissue structure as a therapeutic agent for muscle diseases, such as Duchenne muscular dystrophy, muscular atrophy, and sarcopenia, caused by genetic factors as well as traumatic muscle damage, the MEM-align tissue structure was transplanted into the quadriceps femoris (QF) muscle area of an MDX transgenic mouse model and then a histological analysis was performed 4 weeks after transplantation. As a result of analysis by H&E staining, the number of necrotic fibers and centrally nucleated myofibers was significantly smaller in the MEM-align group than in the NT and MEM-only groups.
(c, d) Masson's trichrome staining also confirmed that the degree of fibrotic infiltration was greatly lower in the MEM-align group than in the NT and MEM-only groups. Also, the collagen-positive area (stained in blue) was also significantly smaller in the MEM-align group than in the NT and MEM-only groups.
(e, f) The largest number of dystrophin-positive myofibers were also observed in the mouse treated with the MEM-align tissue structure. Therefore, it was found that the MEM-align tissue structure was effective in preserving the integrity of muscle cells.
(g, h) As the functionalities of the transplanted artificial muscle tissue, walking ability, balance ability and muscular endurance were assessed for up to 8 weeks by gait analysis and hanging test. Specifically, the hindlimb stance, stride and sway were analyzed and compared with the values before operation. In the MEM-only group, the stance and stride length decreased and the sway length did not change significantly compared with the values before transplantation. However, in the MEM-align group, the stance and stride length were significantly improved and the sway length increased compared with the values before operation. Therefore, this result indicates that the MEM-align group can be effective in delaying and alleviating muscle pathology in the MDX transgenic model (increasing the stance and stride length and maintaining the sway length). Also, according to the hanging test, the mice of the MEM-align group exhibited longer hanging time than the mice of the MEM-only group. Overall, the inspired muscle regeneration ability and the functional recovery of damaged muscle of the artificial muscle tissue produced according to the present disclosure were confirmed through the tests on animal models of two types of muscle damage VML and MDX.

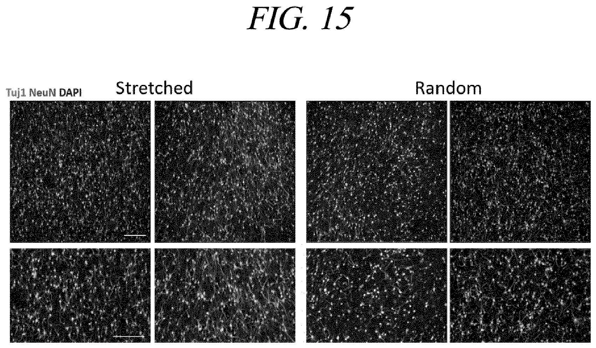

### Example 15: Three-dimensional anisotropic alignment of induced neuronal cells cultured in 3D BEM hydrogel (FIG. 15)

By the method according to the present disclosure, fibrils were aligned in a decellularized brain extracellular matrix (BEM) in a 3D BEM hydrogel, followed by culture of induced neuronal cells. On day 5 of culture, immunostaining of Tuj1 and NeuN, neuron-specific markers, was performed. As a result, it was confirmed that the induced neuronal cells were aligned along the fibrils aligned in one direction. In this case, the induced neuronal cells were produced by a direct reprogramming technology by which three transcription factors, Brn2, Ascl1 and Myt11, were delivered to mouse fetal fibroblasts. This result indicates that the method of the present disclosure can be applied not only to muscle, but also to various tissues such as brain and thus can be applied to various tissue regeneration technologies.

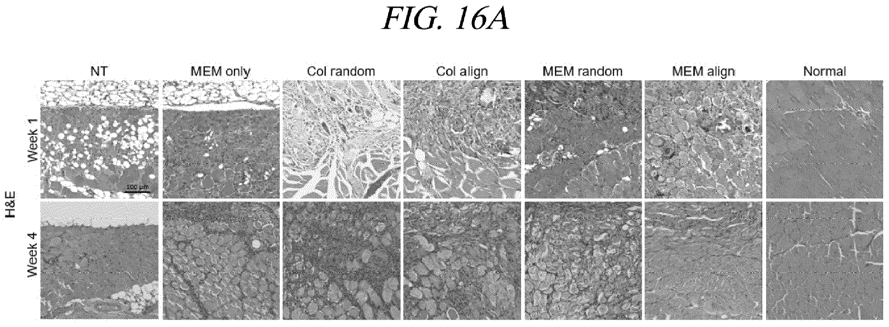

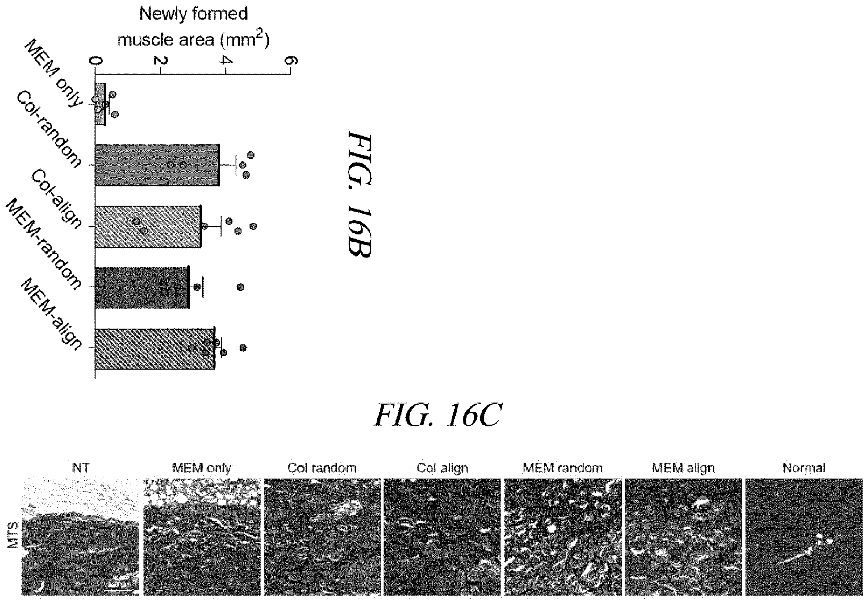

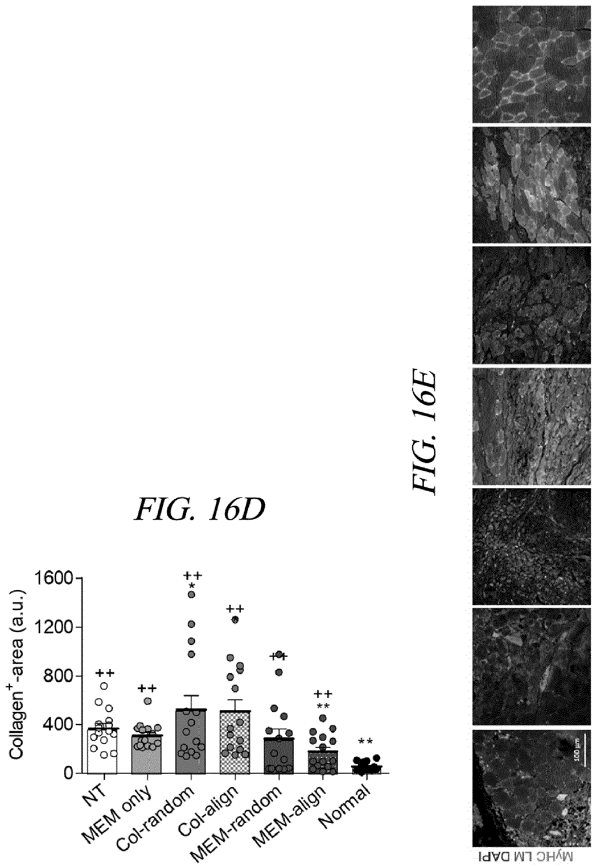

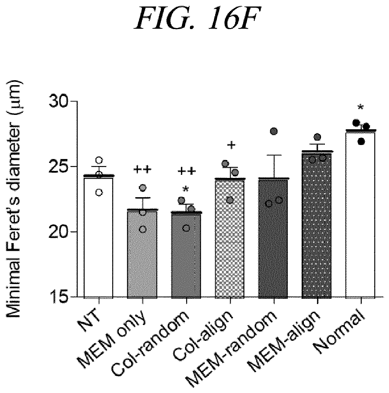

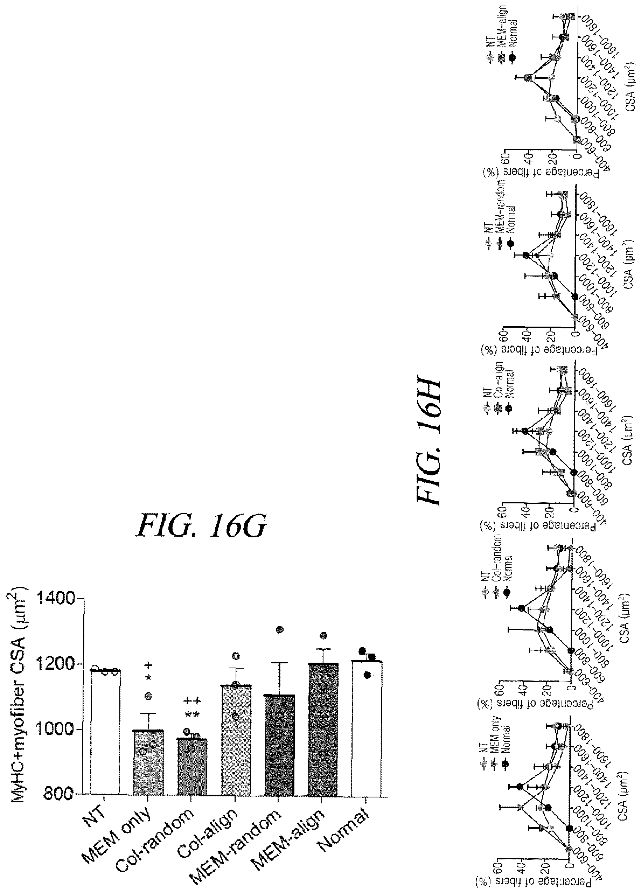

### Example 16: Three-dimensional anisotropic alignment of iMPCs and induced neuronal cells co-cultured in 3D MEM hydrogel (FIG. 16)

By the method according to the present disclosure, fibrils were aligned in an MEM in a 3D MEM hydrogel, followed by co-culture of iMPCs and induced neuronal cells (iN cells) produced by a direct reprogramming technology. It was observed that the neurites of the iN cells were in contact with the iMPCs along the aligned fibrils, which confirmed the possibility of mimicking the neuromuscular junction structure important for muscle function.

## Claims

1. A method of preparing a tissue gel for transplantation, comprising:
a process (a) of preparing a composition containing a decellularized extracellular matrix;
a process (b) of maintaining a tensile state by applying a tensile force to a stretching device made of an elastic polymer material; and
a process (c) of injecting the composition prepared in the process (a) into the stretching device maintained in the tensile state in the process (b), allowing the composition to crosslink, and then removing the tensile force to align fibrils in the decellularized extracellular matrix in one direction.

2. The method of preparing a tissue gel for transplantation of Claim 1,
wherein in the process (a), the decellularized extracellular matrix is derived from one or more tissues selected from the group consisting of muscle, brain, spinal cord, tongue, airway, skin, lymph, lung, heart, liver, stomach, kidney, pancreas, intestine, skeletal muscle, fat, uterus, thymus, esophagus, salivary gland, bone, bladder, blood vessel, tendon, pancreas, thyroid gland, ligament and cartilage.

3. The method of preparing a tissue gel for transplantation of Claim 1,
wherein in the process (a), the decellularized extracellular matrix includes collagen, glycoprotein, proteoglycan, ECM-affiliated protein and ECM modulator, and
the collagen, glycoprotein, proteoglycan, ECM-affiliated protein and ECM modulator are included in an amount of 15 wt% to 45 wt%, 20 wt% to 40 wt%, 10 wt% to 20 wt%, 0.1 wt% to 10 wt% and 10 wt% to 30 wt%, respectively, with respect to a total weight of the decellularized extracellular matrix protein.

4. The method of preparing a tissue gel for transplantation of Claim 1,
wherein the process (a) further includes loading induced skeletal myogenic progenitor cells or induced neuronal cells into the composition.

5. The method of preparing a tissue gel for transplantation of Claim 1,
wherein in the process (b), the stretching device is an elastic material containing polydimethylsiloxane (PDMS).

6. The method of preparing a tissue gel for transplantation of Claim 1,
wherein in the process (b), the tensile force acts in a direction perpendicular to the one direction of the process (c).

7. The method of preparing a tissue gel for transplantation of Claim 4, further comprising:
a process (d) of culturing the loaded induced skeletal myogenic progenitor cells or induced neuronal cells after the tensile force is removed in the process (c).

8. The method of preparing a tissue gel for transplantation of Claim 7,
wherein in the process (d), the culture is performed within 21 days at a temperature of 30°C to 40°C.

9. A tissue gel for transplantation, comprising:
a decellularized extracellular matrix which includes collagen, glycoprotein, proteoglycan, ECM-affiliated protein and ECM modulator and in which the collagen, glycoprotein, proteoglycan, ECM-affiliated protein and ECM modulator are included in an amount of 15 wt% to 45 wt%, 20 wt% to 40 wt%, 10 wt% to 20 wt%, 0.1 wt% to 10 wt% and 10 wt% to 30 wt%, respectively, with respect to a total weight of the decellularized extracellular matrix protein, and fibrils in the decellularized extracellular matrix are aligned in one direction.

10. The tissue gel for transplantation of Claim 9,
wherein the decellularized extracellular matrix is derived from one or more tissues selected from the group consisting of muscle, brain, spinal cord, tongue, airway, skin, lymph, lung, heart, liver, stomach, kidney, pancreas, intestine, skeletal muscle, fat, uterus, thymus, esophagus, salivary gland, bone, bladder, blood vessel, tendon, pancreas, thyroid gland, ligament and cartilage.

11. The tissue gel for transplantation of Claim 9, further comprising:
induced skeletal myogenic progenitor cells or induced neuronal cells loaded and cultured in the decellularized extracellular matrix.

12. The tissue gel for transplantation of Claim 9,
wherein as a result of measuring a storage modulus of the tissue gel for transplantation, the storage modulus is from 300 Pa to 1,000 Pa.
